# EUROPEAN PATENT SPECIFICATION

(11) **EP 3 859 080 B1**
(45) Date of publication and mention of the grant of the patent: **11.10.2023**
(21) Application number: 19882917.8
(22) Date of filing: 07.11.2019
(51) Int. Cl.: D21H 11/18, D21H 11/14, D21C 5/02, D21C 9/00, B09B 3/00, B09B 5/00, B29B 17/02, C08J 11/16

(54) **METHOD FOR MANUFACTURING PULP FIBERS FOR CELLULOSE NANOFIBERIZATION**
VERFAHREN ZUR HERSTELLUNG VON PULPEFASERN FÜR ZELLULOSENANOZERFASERUNG
PROCÉDÉ DE FABRICATION DE FIBRES DE PÂTE À PAPIER POUR NANODÉFIBRAGE DE CELLULOSE

(30) Priority: 09.11.2018 JP 2018211713; 28.12.2018 JP 2018248570
(43) Date of publication of application: 04.08.2021
(73) Proprietor: Unicharm Corporation, Shikokuchuo-shi, Ehime 799-0111 (JP)
(72) Inventor: KONISHI, Takayoshi, Kanonji-shi, Kagawa 769-1602 (JP); HIRAOKA, Toshio, Kanonji-shi, Kagawa 769-1602 (JP); KURITA, Noritomo, Kanonji-shi, Kagawa 769-1602 (JP); BANDOU, Takeshi, Kanonji-shi, Kagawa 769-1602 (JP)
(74) Representative: Dolleymores
(86) International application number: PCT/JP2019/043761
(87) International publication number: WO 2020/096014

(56) References cited:
- EP-A1- 3 617 401
- WO-A1-2015/190140
- WO-A1-2018/025501
- JP-A- 2000 084 533
- JP-A- 2014 176 510
- JP-A- 2017 100 133
- JP-A- 2019 005 733

## Description

### FIELD

The present disclosure relates to a method of producing pulp fibers for cellulose nanofiberization from pulp fibers contained in a used sanitary product.

### BACKGROUND

There are studies of a technology for recycling a used sanitary product such as disposable diapers. For example, Patent Literature 1 discloses a method of producing recycled pulp mainly reusable as a sanitary product. Specifically, Patent Literature 1 discloses a method of collecting pulp fibers from a used sanitary product containing pulp fibers and superabsorbent polymers to produce recycled pulp reusable as a sanitary product, the method including a step of decomposing the used sanitary product into the pulp fibers and other materials by exerting a physical force to the used sanitary product in an aqueous solution containing polyvalent metal ions or an acidic aqueous solution having pH of 2.5 or less, a step of separating the pulp fibers from a mixture of the pulp fibers and the other materials generated in the decomposition step, and a step of treating the separated pulp fibers in an ozone-containing aqueous solution having pH of 2.5 or less.

### [CITATION LIST]

### [PATENT LITERATURE]

Patent Literature 1: Japanese Unexamined Patent Publication No.2016-881.
EP 3626882 A1 falls under Article 54(3) EPC and relates to a method for producing pulp fibers for cellulose nanofiberization from pulp fibers of used sanitary products.
WO 2015/190140 A1 relates to a method for manufacturing recycled pulp from used sanitary products.
EP 3617401 A1 falls under Article 54(3) EPC and relates to a method for producing pulp fibres for saccharification from the pulp fibres of used sanitary items, said pulp fibres for saccharification having low lignin contents distributed within a narrow range.

### SUMMARY

### [TECHNICAL PROBLEM]

Patent Literature 1 does not disclose that "recycled pulp fibers "are reused as "pulp fibers for cellulose nanofiberization". Therefore, an object of the present disclosure is to provide a method of producing pulp fibers for cellulose nanofiberization from pulp fibers contained in a used sanitary product.

### [SOLUTION TO PROBLEM]

The inventors have found a method, as claimed in claim 1, of producing pulp fibers for cellulose nanofiberization from pulp fibers contained in a used sanitary product. The present invention relates to a method of producing pulp fibers for cellulose nanofiberization from pulp fibers contained in a used sanitary product, as claimed in any one of claims 1 to 9. The dependent claims specify preferred but optional features.

### [ADVANTAGEOUS EFFECT OF THE INVENTION]

The production method of the present disclosure can produce pulp fibers for cellulose nanofiberization from the pulp fibers contained in the used sanitary product.

### BRIEF DESCRIPTION OF DRAWINGS

[Fig. 1]

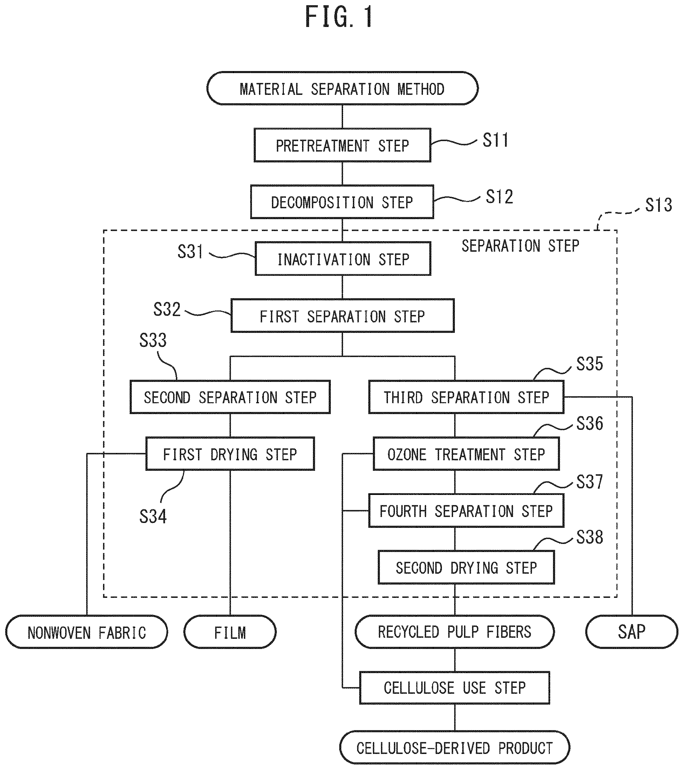

   Fig. 1 is a flow chart showing an embodiment according to the method of the present disclosure.
[Fig. 2]

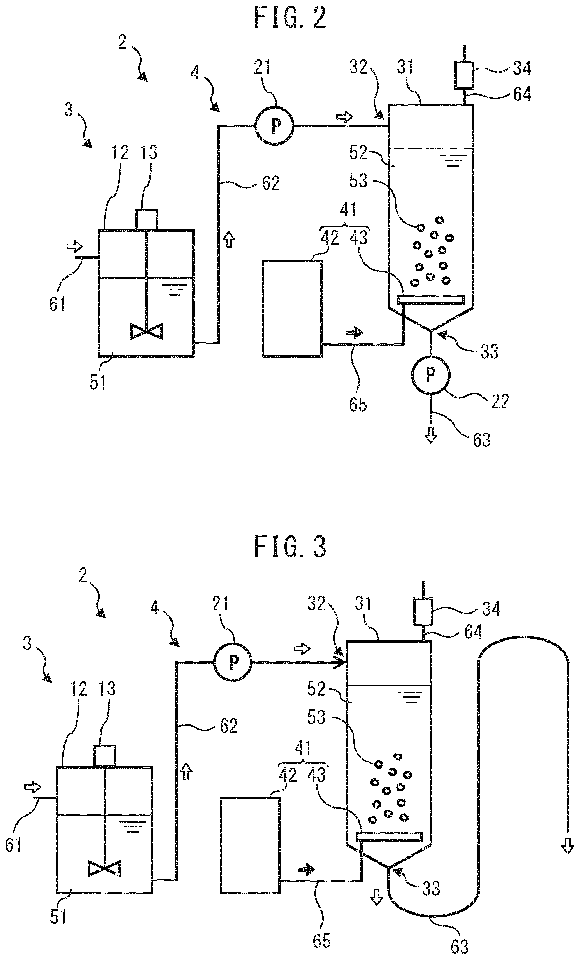

   Fig. 2 is a schematic view showing a reference example of an apparatus of an ozone treatment step.
[Fig. 3]
   Fig. 3 is a schematic view showing another reference example of an apparatus of an ozone treatment step.
[Fig. 4]

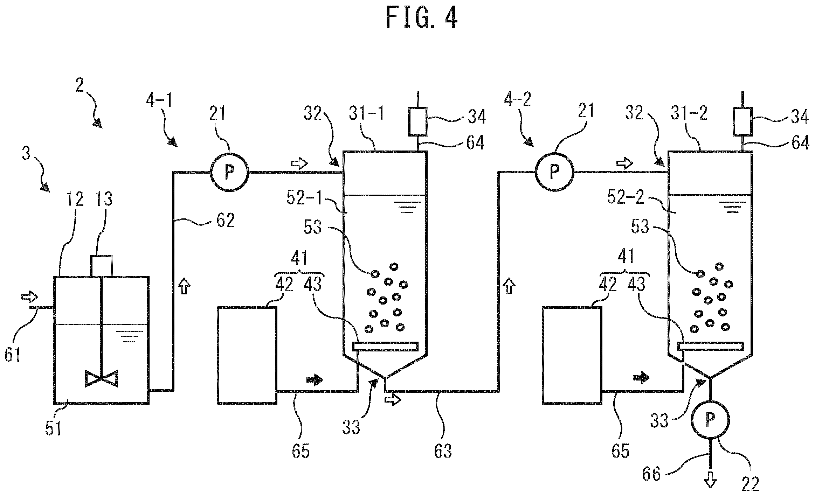

   Fig. 4 is a schematic view showing still another reference example of an apparatus of an ozone treatment step.

### DESCRIPTION OF EMBODIMENTS

The present disclosure specifically relates to the following aspects.

### [Aspect 1]

A method of producing pulp fibers for cellulose nanofiberization from pulp fibers contained in a used sanitary product, the method comprising: pulp fibers for cellulose nanofiberization forming step of forming the pulp fibers for cellulose nanofiberization having a lignin content ratio of 0.1 mass % or less from the pulp fibers, by supplying an ozone-containing gas to a treatment tank containing a treatment liquid containing pulp fibers-containing material containing superabsorbent polymers and pulp fibers which derive from the used sanitary product.

In the production method described above, the pulp fibers-containing material containing the superabsorbent polymers and the pulp fibers derived from the used sanitary product is brought into contact with the ozone-containing gas. The ozone-containing gas decomposes the superabsorbent polymers and decomposes lignin in the pulp fibers to reduce a lignin content ratio in the pulp fibers to a predetermined value. Accordingly, in the production method described above, it is possible to produce pulp fibers for cellulose nanofiberization which are excellent in cellulose nanofiber formation. Incidentally, recycled pulp fibers having a high lignin content ratio tend to be difficult to form cellulose nanofibers.

The method further comprises: a preparation step of preparing the treatment tank including pulp fibers-containing material supply port, a treatment liquid discharge port, and an ozone-containing gas supply port arranged at a lower position of the treatment tank, pulp fibers-containing material supply step of supplying the pulp fibers-containing material to the treatment tank from the pulp fibers-containing material supply port, an ozone-containing gas supply step of supplying the ozone-containing gas to the treatment liquid in the treatment tank from the ozone-containing gas supply port, the pulp fibers for cellulose nanofiberization forming step of forming the pulp fibers for cellulose nanofiberization from the pulp fibers, as well as bringing the pulp fibers-containing material into contact with the ozone-containing gas while raising the ozone-containing gas in the treatment tank, to dissolve at least a part of the superabsorbent polymers in the treatment liquid, and a treatment liquid discharge step of discharging the treatment liquid containing the pulp fibers for cellulose nanofiberization from the treatment liquid discharge port.

In the used sanitary product, in an absorbent body, etc., containing pulp fibers and superabsorbent polymers, (i) the superabsorbent polymers enlarge as they absorb a liquid such as a bodily fluid and entrains the pulp fibers, (ii) the enlarged superabsorbent polymers generate gel blocking while entraining the pulp fibers,etc., and accordingly, the plurality of superabsorbent polymers and the plurality of pulp fibers may form a coupling structure.

In the method disclosed in Patent Literature 1, the ozone-containing gas can treat free pulp fibers that do not form a coupling structure and decompose lignin contained in the pulp fibers, but is difficult to come into contact with the pulp fibers configuring the inside of the coupling structure, that is, the pulp fibers surrounded by the superabsorbent polymers, and accordingly, the pulp fibers constituting the coupling structure cannot be sufficiently treated, that is, the lignin contained in the pulp fibers surrounded by superabsorbent polymers may be difficult to be decomposed.

Therefore, the recycled pulp fibers produced by the method disclosed in Patent Literature 1 had a wide distribution of lignin content ratio and included those having a high lignin content ratio. In addition, the distribution of the lignin content ratio tended to be wider, in a case where the used sanitary product used as a raw material includes a plurality of types thereof, for example, includes the same type of products sold by different companies (disposable diapers for adults) and different types of products sold by the same company (for example, disposable diapers for adults and disposable diapers for children).

The production method includes a predetermined pulp fibers for cellulose nanofiberization forming step, and in the pulp fibers for cellulose nanofiberization forming step described above, the superabsorbent polymers and pulp fibers are brought into contact with the ozone-containing gas. In the free superabsorbent polymers, the free pulp fibers, and the coupling structure, the free superabsorbent polymers and coupling structure having a relatively low buoyancy tend to have higher sedimentation properties than the free pulp fibers having a relatively high buoyancy. Meanwhile, the ozone-containing gas rises while consuming ozone and treating the superabsorbent polymers and the pulp fibers, and accordingly, the ozone-containing gas existing at the lower position has a higher ozone content ratio than the ozone-containing gas existing at an upper position (that is, it tends to be fresh).

In the present specification, a falling-down speed is related to a downward movement speed of the treatment liquid in the treatment tank, and is generally uniquely determined by a first flow rate, a second flow rate, a size of the treatment tank, and the like. Meanwhile, in the present specification, the sedimentation properties mean properties of the pulp fibers, the superabsorbent polymers, and the coupling structure contained in the treatment liquid in the treatment tank representing ease of falling down in a vertical direction due to gravity, and the pulp fibers, the superabsorbent polymers, and each of the pulp fibers, the superabsorbent polymers, and the coupling structure have different sedimentation properties depending on the specific gravity and the like.

Therefore, in the production method described above, the free superabsorbent polymers and the superabsorbent polymers in the coupling structure having a relatively high sedimentation properties are accurately oxidatively decomposed by the fresher ozone-containing gas to free the pulp fibers configuring the coupling structure, and the free pulp fibers having a relatively low sedimentation properties can be treated by the ozone-containing gas by taking time to decompose lignin contained in the free pulp fibers.

In general, the higher the lignin content ratio of pulp fibers, the lower the specific gravity tends to be. Therefore, in the production method described above, the pulp fibers having a relatively low lignin content ratio have a relatively high sedimentation properties than the pulp fibers having a relatively high lignin content ratio, and accordingly, the fresher ozone-containing gas can come into contact with the pulp fibers having a relatively low lignin content ratio to further decompose the lignin contained therein.

In the production method described above, the pulp fibers for cellulose nanofiberization produced by the production method described above does not easily expand the distribution of the lignin content ratio, and the pulp fibers for cellulose nanofiberization further have a predetermined lignin content ratio. Accordingly, in the production method described above, it is possible to produce pulp fibers for cellulose nanofiberization which have a narrow distribution of the lignin content ratio and are excellent in cellulose nanofiber formation.

The ozone-containing gas supply port means a lower position of the treatment tank, specifically, a range of preferably 30%, more preferably 20%, and even more preferably 10% of a height of the treatment tank from a bottom of the treatment tank. In addition, the pulp fibers-containing material supply port may be arranged at a lower side or an upper side of the ozone-containing gas supply port, and the treatment liquid discharge port may be arranged at a lower side or an upper side of the ozone-containing gas supply port.

The production method according to the aspect 2 includes both so-called continuous production method and batch production method. In addition, the "raising the ozone-containing gas" means that the ozone-containing gas is rising as a whole, and includes the raising of the ozone-containing gas as a whole in a vertical direction while being stirred in a horizontal direction, when the treatment liquid in the treatment tank is stirred.

The treatment liquid discharge port is arranged on an upper side of the pulp fibers-containing material supply port, and in the pulp fibers for cellulose nanofiberization forming step, the pulp fibers-containing material is brought into contact with the ozone-containing gas while raising the pulp fibers-containing material.

In the production method described above, the rising pulp fibers-containing material is brought into contact with the rising ozone-containing gas, and accordingly, the pulp fibers-containing material is in contact with the ozone-containing gas for a longer time, and the hemicellulose content ratio in the pulp fibers is lowered.

The production method according to the present invention includes both so-called continuous production method and batch production method. In addition, the "raising the pulp fibers-containing material" means that at least a part of the pulp fibers-containing material rises, for example, the entirety thereof when the entire treatment liquid in the treatment tank rises and a part of the pulp fibers-containing material rising when the treatment liquid in the treatment tank is convected vertically may be included.

### [Aspect 2]

The method according to aspect 1, further comprising an inactivation step of inactivating the superabsorbent polymers with an acid before the pulp fibers-containing material supply step.

Since the production method described above further includes the predetermined inactivation step, even when the superabsorbent polymers and the pulp fibers form the coupling structure, the ozone in the ozone-containing gas can remove the superabsorbent polymers constituting the coupling structure immediately after the pulp fibers-containing material is supplied to the treatment tank, and the ozone in the ozone-containing gas can act on the pulp fibers constituting the coupling structure to decrease the lignin content ratio of the pulp fibers (form the pulp fibers for cellulose nanofiberization having a low lignin content ratio).

### [Aspect 3]

The method according to the aspect 2, wherein the acid is an acid capable of forming a complex with a metal ion contained in excrement.

In the production method described above, since the acid is an acid capable of forming a complex with a metal ion contained in excrement, the produced pulp fibers for cellulose nanofiberization are less likely to contain the metal ions. Therefore, in a case of producing cellulose nanofibers from the pulp fibers for cellulose nanofiberization, metal ions and their precipitates are less likely to damage equipment used in cellulose nanofibers forming step, and it is difficult to inhibit miniaturization of the pulp fibers for cellulose nanofiberization.

### [Aspect 4]

The method according to any one of the aspects 1 to 3 wherein, in the pulp fibers-containing material supply step, the pulp fibers-containing material is continuously supplied from the pulp fibers-containing material supply port to the treatment tank at a first flow rate, and in the treatment liquid discharge step, the treatment liquid is continuously discharged from the treatment liquid discharge port at a second flow rate.

In the production method described above, the pulp fibers-containing material is continuously supplied from the pulp fibers-containing material supply port to the treatment tank at the first flow rate in the pulp fibers-containing material supply step, and the treatment liquid is continuously discharged from the treatment liquid discharge port at the second flow rate in the treatment liquid discharge step, and accordingly, the treatment time of the superabsorbent polymers and the pulp fibers to be treated is uniform, and the distribution of the lignin content ratio of the pulp fibers for cellulose nanofiberization contained in the treatment liquid is narrowed (variation is reduced). Accordingly, in the production method described above, it is possible to produce pulp fibers for cellulose nanofiberization which are excellent in cellulose nanofiber formation.

### [Aspect 5]

The method according to any one of the aspects 1 to 4, wherein the treatment liquid is acidic, weakly acidic, or neutral.

In the production method described above, the treatment liquid is acidic (specifically, pH of more than 0.0 and pH of less than 3.0, preferably pH of 2.5 or more and pH of less than 3.0), weakly acidic (specifically, pH of 3.0 or more and pH of less than 6.0), or neutral (specifically, pH of 6.0 or more and pH of 8.0 or less, preferably pH of 6.0 or more and pH of 7.0 or less). Accordingly, the superabsorbent polymers to be treated can be inactivated by acid, or if the superabsorbent polymers to be treated are already inactivated, the superabsorbent polymers can be continuously maintained in the inactivated state. Due to that, even when the superabsorbent polymers and the pulp fibers form the coupling structure, the ozone in the ozone-containing gas can remove the superabsorbent polymers constituting the coupling structure, and the ozone in the ozone-containing gas can act on the pulp fibers constituting the coupling structure to decrease the lignin content ratio of the pulp fibers (form the pulp fibers for cellulose nanofiberization having a low lignin content ratio).

### [Aspect 6]

The method according to any one of the aspects 1 to 5, wherein , in the pulp fibers for cellulose nanofiberization forming step, the ozone-containing gas is supplied as microbubbles or nanobubbles.

In the production method described above, in the pulp fibers for cellulose nanofiberization forming step, the ozone-containing gas is supplied as microbubbles or nanobubbles from the ozone-containing gas supply port, and accordingly, even in a case where the superabsorbent polymers and the pulp fibers form a coupling structure, the microbubbles or nanobubbles apply buoyancy to the superabsorbent polymers, the coupling structure, and pulp fibers, thereby reducing the sedimentation properties thereof. Therefore, it takes a longer time for the superabsorbent polymers, the coupling structure, and the pulp fibers to reach the treatment liquid discharge port, the ozone can decompose the free superabsorbent polymers and the superabsorbent polymers constituting the coupling structure, and the free pulp fibers and the pulp fibers constituting the coupling structure can be sufficiently treated. Accordingly, in the production method described above, it is possible to produce pulp fibers for cellulose nanofiberization which are excellent in cellulose nanofiber formation.

### [Aspect 7]

The method according to any one of aspects 1 to 6, further comprising cellulose nanofibers forming step of forming cellulose nanofibers from the pulp fibers for cellulose nanofiberization, after the pulp fibers for cellulose nanofiberization forming step.

In the production method described above, it is possible to efficiently produce cellulose nanofibers from the pulp fibers for cellulose nanofiberization.

### [Aspect 8]

The method according to any one of aspects 1 to 7, wherein the pulp fibers for cellulose nanofiberization have a beating degree reduction rate of 300 mL/h or more.

In the production method described above, since the pulp fibers for cellulose nanofiberization have a predetermined beating degree reduction rate, the pulp fibers for cellulose nanofiberization are easily fluffed and easily converted into cellulose nanofibers in the subsequent cellulose nanofibers forming step. Accordingly, in the production method described above, it is possible to produce pulp fibers for cellulose nanofiberization which are excellent in cellulose nanofiber formation.

### [Aspect 9]

The method according to any one of aspects 1 to 8, wherein the pulp fibers for cellulose nanofiberization have an ash content of 0.65 mass % or less.

In the production method described above, the pulp fibers for cellulose nanofiberization produced by the production method described above have a predetermined ash content. Therefore, in a case of producing cellulose nanofibers from the pulp fibers for cellulose nanofiberization, metal ions and their precipitates are less likely to damage equipment used in cellulose nanofibers forming step, and it is difficult to inhibit miniaturization of the pulp fibers for cellulose nanofiberization.

Hereinafter, a method of producing pulp fibers for cellulose nanofiberization from pulp fibers of a used sanitary product (hereinafter, may be simply referred to as a "method of producing pulp fibers for cellulose nanofiberization") will be described. The used sanitary product described above is a sanitary product used by a user and includes a sanitary product in a state where the user's liquid excrement is absorbed, and includes a sanitary product which is used but has not absorbed excrement, a sanitary product which is not used, and the like.

First, a configuration example of the sanitary product will be described. The sanitary product includes a top-surface sheet, a back-surface sheet, an absorbent body arranged between the top-surface sheet and the back-surface sheet. Examples of a sanitary product include a paper diaper, a urine absorbing pad, a sanitary napkin, a bed sheet, and a pet sheet.

Examples of a constituent member of the top-surface sheet include a nonwoven fabric or a film, and specific examples thereof include a liquid-permeable nonwoven fabric, a synthetic resin film having liquid-permeable holes, and a composite sheet thereof. Examples of a constituent member of the back-surface sheet include a nonwoven fabric or a film, and specific examples thereof include a liquid-impermeable nonwoven fabric, a liquid-impermeable synthetic resin film, and a composite sheet of these nonwoven fabric and the synthetic resin film.

Examples of a constituent members of the absorbent body include an absorbent core (for example, pulp fibers and superabsorbent polymers) and a core wrap. The pulp fibers are not particularly limited as long as they can be used as a sanitary product, and examples thereof include a cellulosic fiber. Examples of the cellulosic fiber include a wood pulp, a crosslinked pulp, and a non-wood pulp, a regenerated cellulose, a semi-synthetic cellulose, and the like. The superabsorbent polymers (SAP) are not particularly limited as long as they can be used as a sanitary product, and examples thereof include polyacrylate-based, polysulfonate-based, and maleic anhydride-based polymers.

One surface and the other surface of the absorbent body are bonded to the top-surface sheet and the back-surface sheet, respectively, via an adhesive. In a plan view, a portion (peripheral portion) of the top-surface sheet which extends to an outer side of the absorbent body so as to surround the absorbent body is bonded to a portion (peripheral portion) of the back-surface sheet which extends to an outer side of the absorbent body to surround the absorbent body via an adhesive. Accordingly, the absorbent body is wrapped inside the bonded body of the top-surface sheet and the back-surface sheet. The adhesive is not particularly limited as long as it can be used as a sanitary product and a bonding force is lowered by softening or the like due to hot water which will be described later, and examples thereof include a hot-melt type adhesive. Examples of the hot-melt type adhesive include a pressure-sensitive adhesive or a heat-sensitive adhesive of a rubber-based entity such as styrene-ethylene-butadiene-styrene, styrene-butadiene-styrene, and styrene-isoprene-styrene, or an olefin-based entity such as polyethylene.

Fig. 1 is a flow chart showing a material separation method of separating the used sanitary product into constituent materials. This material separation method is a method of separating the used sanitary product into a film, a nonwoven fabric, pulp fibers, and superabsorbent polymers. This material separation method includes a pretreatment step S 11, a decomposition step S12, and a separation step S13. In the pretreatment step S11, the used sanitary product is swollen with water. In the decomposition step S12, a physical impact is applied to the swollen used sanitary product, and the used sanitary product is decomposed into a film, a nonwoven fabric, and a core wrap, and absorbent core (for example, pulp fibers and superabsorbent polymers). In the separation step S13, the film, the nonwoven fabric, the pulp fibers, and the superabsorbent polymers are separated.

The method of producing the pulp fibers for cellulose nanofiberization of the present disclosure is included in the separation step S13 of this material separation method. If a mixture of the pulp fibers and the superabsorbent polymers has been obtained in advance by any method, it is practically not necessary to perform the steps earlier than the method of producing the pulp fibers for cellulose nanofiberization in the pretreatment step S 11, the decomposition step S12, and the separation step S13. Hereinafter, each step will be described.

In the pretreatment step S 11, a plurality of used sanitary products absorb water to be swollen, in a state where the sanitary products are collected from outside, that is, in a rolled state or a folded state without being destructed or cut, without inactivation of the superabsorbent polymers of the absorbent body. In the present embodiment, the used sanitary products are made to absorb hot water and swell, or made to absorb water to be expanded and then the absorbed water is heated to be hot water. The hot water refers to water having a temperature higher than room temperature (20°C ± 15°C (5°C to 35°C): JISZ8703).

Normally, an amount of liquid excrement actually absorbed by the used sanitary product is extremely small, compared to a maximum absorption amount that sanitary products can absorb (for example, approximately 10 to 20 mass % of the maximum absorption amount). In the present embodiment, in the pretreatment step S11, by immersing the used sanitary product in hot water, an amount of water close to the maximum absorption amount of the used sanitary product (for example, 80 mass % or more of the maximum absorption amount) is absorbed. Alternatively, the used sanitary product is immersed in water at room temperature to absorb water to an amount close to the maximum absorption amount of the used sanitary product, and then the entire used sanitary product is heated to a temperature of hot water. Due to that, the used sanitary product can be brought into an extremely expanded state with hot water or water at room temperature (hereinafter, also simply referred to as "hot water"). Due to that, in the used sanitary product, an extremely high internal pressure is generated. In addition, an object of turning the water into hot water is mainly to weaken the adhesive strength of the adhesive as will be described later.

Here, in a case where the used sanitary product is initially immersed in hot water when it is rolled or folded with the back-surface sheet on the outside (the top-surface sheet is hidden inside), and accordingly, the absorbent body of the used sanitary product absorbs hot water in the hot water and expands. Due to that, the internal pressure of the used sanitary product increases, and a force to open the used sanitary product outward is exerted to the used sanitary product, so that the used sanitary product in the rolled or folded state is opened outward and is in a substantially flat state. That is, the used sanitary product can be unfolded to be flattened in hot water. At this time, since the absorbent body of the used sanitary product absorbs a large amount of hot water and expands extremely, the surface thereof, that is, any portion of the top-surface sheet and the back-surface sheet wrapping the absorbent body is likely to be easily burst. That is, in the pretreatment step S 11, any surface of the used sanitary product can be in a state where it is likely to be torn and cut. In a case where the used sanitary product is in an unfolded state to be flattened from the beginning, any portion of the surface is likely to be easily burst as it is. This state cannot occur, in a case where the used sanitary product is broken or the like.

In addition, the used sanitary product is immersed in hot water and/or absorbs the hot water to soften an adhesive (for example, hot-melt adhesive) used for bonding constituent members to each other by the heat of the hot water, thereby reducing a bonding force of the adhesive. For example, the adhesive for bonding the peripheral portion of the top-surface sheet and the peripheral portion of the back-surface sheet to each other can be softened by the heat of hot water to reduce the bonding force of the adhesive. In addition, the adhesive for bonding the top-surface sheet and the absorbent body and the adhesive for bonding the back-surface sheet and the absorbent body can be softened by the heat of hot water to reduce the bonding force of the adhesives.

As described above, in the pretreatment step S11, the expansion of the absorbent body of the used sanitary product can generate a state where any portion of the surface of the used sanitary product is likely to burst, and a state where the bonding force of the adhesive is reduced. By setting the used sanitary product in such a state, the used sanitary product can be reliably decomposed in the decomposition step which will be described later.

A temperature of the hot water in the pretreatment step S11 is not particularly limited as long as the adhesive of the used sanitary product can be softened, and for example, it is 60°C or higher and preferably 70°C or higher and 98°C or lower. By setting the temperature of the hot water to 70°C or higher, the adhesive for bonding the constituent members to each other can be further softened by the heat of the hot water, and the bonding force of the adhesive can be further reduced. By setting the temperature of the hot water to 98°C or lower, the hot water certainly exists as a liquid, and accordingly, the used sanitary product can more reliably absorb the hot water. Due to the expansion of the absorbent body and the heat of the hot water, it is possible to more reliably generate a state in which the surface of the used sanitary product is likely to burst and a state in which the bonding force of the adhesive is reduced. For temperature measurement, a temperature of hot water in which the used sanitary product is immersed is measured, or a temperature of an inner part of 5 mm from the surface of the used sanitary product which has absorbed water to an amount close to the maximum absorption amount (tip of a temperature sensor is inserted) is measured.

In addition, the sterilization of constituent materials is extremely important in the reuse of the used sanitary product. It is preferable that the temperature of the hot water is 70°C or higher, because it is possible to exhibit an effect of sterilizing (disinfecting) the used sanitary product.

The treatment time in the pretreatment step S11, that is, the time for immersing the used sanitary product in hot water is not particularly limited as long as the absorbent body of the used sanitary product can expand, and is, for example, 2 to 60 minutes and preferably 4 to 30 minutes. In a case where the time is extremely short, the absorbent body cannot expand sufficiently, and in a case where the time is extremely long, the time is wasted and the treatment cost increases unnecessarily.

The amount of hot water absorbed by the absorbent body in the pretreatment step S11 is not particularly limited as long as the absorbent body can expand to the extent that the used sanitary product can be decomposed in the decomposition step which will be described later, and is, for example, 80 mass % or more of the maximum absorption amount of the used sanitary product and is preferably 90 mass % or more. Due to that, the used sanitary product can be fully expanded with water. As a result, an extremely high internal pressure can be generated in the absorbent body of the used sanitary product.

It should be noted that the maximum absorption amount is measured by the following procedure.
(1) An unused sanitary product is dried in an atmosphere at 100°C or higher, and the mass of the sanitary product is measured.
(2) In a case where a stretchable material (for example, a stretchable member around the legs, around the waist, or the like) that can form a pocket that makes it difficult for water to reach the absorbent body is arranged in the sanitary product, a cut is made in the stretchable member to flatten the sanitary product.
(3) The sanitary product is immersed in a water bath filled with sufficient tap water with the top-surface sheet facing down, and left for 30 minutes.
(4) After being left, the sanitary product is placed on a net with the top-surface sheet facing down and drained for 20 minutes, and then the mass of the sanitary product is measured.

Then, a mass difference before and after the immersion in tap water is defined as the maximum absorption amount.

Next, in the decomposition step S12, a physical impact is applied to the plurality of used sanitary products unfolded and swollen by the pretreatment step S 11 to decompose the plurality of used sanitary product into a film (back-surface sheet), a nonwoven fabric (top-surface sheet), and a core wrap, and an absorbent core (for example, an absorbent body and superabsorbent polymers).

The used sanitary product is unfolded and flat by the pretreatment step S11, and any portion of the surface is likely to burst due to expansion. In the present embodiment, the bonding force of the adhesive is particularly reduced due to the heat of hot water. Accordingly, in the decomposition step S12, by applying a physical impact to the used sanitary product in that state, among any portion of the surface, a bonding portion between the top-surface sheet (nonwoven fabric) and the back-surface sheet (film), where the bonding force is particularly reduced, burst. Due to that, the bonding portion can be torn (peeled). The physical impact is not particularly limited, and for example, a method of throwing the used sanitary product on a surface made of a material harder than the used sanitary product, a method of causing the used sanitary product to be sandwiched and pass between a pair of rolls arranged to face each other and pressing the used sanitary product from both sides, and the like.

In the present embodiment, the decomposition step S12 includes a step of putting the plurality of swollen used sanitary products into a bottom portion of a rotating drum having a horizontal rotating shaft, and a step of rotating the rotating drum around the rotating shaft, pulling the plurality of used sanitary products to the upper part of the rotating drum and throwing them to the bottom portion. Due to that, a physical impact can be stably, continuously (consecutively), and easily applied to the plurality of used sanitary products. As the rotating drum, a rotating drum of a washing tub of a horizontal washing machine is used, for example, and accordingly, the decomposition step S12 can be carried out using a horizontal washing machine of the related art (for example, ECO-22B manufactured by Inamoto Manufacturing Co., Ltd.). A size of the rotating drum is not particularly limited as long as the impact described above can be realized, and an inner diameter and a depth are, for example, 50 to 150 cm and 30 to 120 cm. A rotation speed of the rotating drum is not particularly limited as long as the impact described above can be realized, and is, for example, 30 times/min to 100 times/min.

In addition, a temperature of the used sanitary product is kept relatively high by the hot water absorbed in the used sanitary product, but from a viewpoint of suppressing a temperature drop of the adhesive and maintaining the sterilizing effect, the temperature of the atmosphere in the rotating drum is preferably 70°C or higher and more preferably 75°C or higher. The temperature in the rotating drum is preferably 98°C or lower and more preferably 90°C or lower, from a viewpoint of handling the used sanitary product. It is preferable that the amount of water in the rotating drum is as small as possible, and that the amount of used sanitary product at least at the bottom portion is so small that it does not fall below the water surface. In a case where the used sanitary product is below the water surface, the impact on the used sanitary product is absorbed by the water, and it is difficult to apply the desired impact to the used sanitary product. The time for rotating the rotating drum is not particularly limited as long as the top-surface sheet, the back-surface sheet, the core wrap, and the like and the absorbent core can be decomposed, and is, for example, 2 to 40 minutes and preferably 4 to 20 minutes.

In used sanitary product, the bonding portion between the top-surface sheet (nonwoven fabric) and the back-surface sheet (film) burst and are torn off by the physical impact. At the same time, through a crevice, the internal pressure of the absorbent body causes the absorbent core (for example, pulp fibers and superabsorbent polymers) in the used sanitary product to spurt out (burst out). Due to that, the used sanitary product can be more reliably decomposed into the top-surface sheet (nonwoven fabric), the back-surface sheet (film), and the core wrap, and the absorbent core (for example, pulp fibers and superabsorbent polymers).

Next, in the separation step S13, the plurality of films (back-surface sheets), the plurality of nonwoven fabrics (top-surface sheets), the core wrap, and the like are separated from the absorbent core (for example, pulp fibers and superabsorbent polymers). However, the nonwoven fabric may remain bonded to the film. The separation method is not particularly limited, and examples thereof include a method of using a sieve that allows the absorbent core to pass through without passing through the top-surface sheet, the back-surface sheet, the core wrap, or the like.

In the present embodiment, the separation step S13 includes an inactivation step S31 of inactivating the superabsorbent polymers with an aqueous solution containing an inactivating agent before separating the film, the nonwoven fabric, and the core wrap, from the absorbent core, and a first separation step S32 of separating the film and the nonwoven fabric from the pulp fibers, the inactivated superabsorbent polymers, and a mixture containing wastewater discharged from the superabsorbent polymers by the inactivation.

In the inactivation step S31, before the first separation step S32, the top-surface sheet (nonwoven fabric), the back-surface sheet (film), and the absorbent body (pulp fibers and superabsorbent polymers) are immersed in an aqueous solution containing an inactivating agent capable of inactivating the superabsorbent polymers. Due to that, the superabsorbent polymers attached to the top-surface sheet, the back-surface sheet, and the pulp fibers can be inactivated. Due to that, the superabsorbent polymers in a high viscosity state before the inactivation can be made into superabsorbent polymers in a low viscosity state by dehydration by the inactivation.

Here, the inactivating agent is not particularly limited, and examples thereof include an acid (for example, inorganic acid and organic acid), lime, calcium chloride, magnesium sulfate, magnesium chloride, aluminum sulfate, aluminum chloride, and the like. The acid is preferable because it does not leave ash content in the pulp fibers. In a case where the acid is used as the inactivating agent, the pH is preferably 2.5 or less and more preferably 1.3 to 2.4. In a case where the pH is extremely high, water absorption capacity of the superabsorbent polymers cannot be sufficiently reduced. In addition, the sterilizing ability may be reduced. In a case where the pH is extremely low, there is a risk of equipment corrosion, and a large amount of alkaline chemicals are required for neutralization treatment during sewage treatment.

Examples of the inorganic acid include a sulfuric acid, a hydrochloric acid, and a nitric acid, and the sulfuric acid is preferable from a viewpoint of not containing chlorine and cost. Meanwhile, examples of the organic acid include a citric acid, a tartaric acid, a glycolic acid, a malic acid, a succinic acid, an acetic acid, and an ascorbic acid, and an acid capable of forming a complex with a metal ion contained in excrement, for example, a hydroxycarbonate-based organic acid such as a citric acid, a tartaric acid, and a gluconic acid are particularly preferable. Examples of metal ions contained in excrement include calcium ions. This is because the metal ions in the excrement can be trapped and removed by a chelating effect of the acid capable of forming a complex with the metal ions contained in the excrement. In addition, the citric acid can be expected to have a high dirt component removing effect due to its cleaning effect. Since the pH changes depending on the water temperature, the pH in the present disclosure refers to pH measured at an aqueous solution temperature of 20°C.

A treatment temperature of the inactivation step S31, that is, a temperature of the aqueous solution containing the inactivating agent is not particularly limited as long as the inactivating reaction proceeds. The treatment temperature may be room temperature or higher than room temperature, and is, for example 15 to 30°C. In addition, a treatment time of the inactivation step S31, that is, a time for immersing the top-surface sheet, the back-surface sheet, and the absorbent body in the aqueous solution containing the inactivating agent is not particularly limited as long as the superabsorbent polymers are inactivated and dehydrated, and is, for example, 2 to 60 minutes and preferably 5 to 30 minutes. In addition, an amount of the aqueous solution in the inactivation step S31, that is, an amount of the aqueous solution containing the inactivating agent is not particularly limited as long as the inactivating reaction proceeds. The amount of the aqueous solution is, for example, preferably 300 to 3,000 parts by mass, more preferably 500 to 2,500 parts by mass, and even more preferably 1,000 to 2,000 parts by mass with respect to 100 parts by mass of the used sanitary product.

In the first separation step S32, the top-surface sheet (nonwoven fabric), the back-surface sheet (film), and the core wrap are separated from the pulp fibers, the inactivated superabsorbent polymers, and the mixture containing the wastewater discharged from the superabsorbent polymers due to the inactivation. Note that, the wastewater is wastewater containing water released from the superabsorbent polymers by dehydration with the aqueous solution containing the inactivating agent in the inactivation step S31, that is, liquid derived from excrement and water derived from hot water.

In the first separation step S32, the method of separating the top-surface sheet and the back-surface sheet from the pulp fibers, the superabsorbent polymers, and the wastewater is not particularly limited. For example, a product produced by the inactivation step (the top-surface sheet, the back-surface sheet, the pulp fibers, the superabsorbent polymers, the wastewater, and the like) is discharged while passing through a screen having an opening of 5 to 100 mm, preferably an opening of 10 to 60 mm. Due to that, the product can be separated by remaining the pulp fibers, the superabsorbent polymers, and the wastewater in the sewage, and remaining the top-surface sheet and the back-surface sheet on the screen. In addition, other large-shaped materials such as nonwoven fabric and the films, and the like, may remain on the screen. In particular, since the superabsorbent polymers are in a high viscosity state before inactivation, it is not easy to separate the superabsorbent polymers attached to the top-surface sheet, the back-surface sheet, and the pulp fibers. However, after the inactivation, the superabsorbent polymers have low viscosity due to dehydration. Therefore, the superabsorbent polymers attached to the top-surface sheet, the back-surface sheet, and the pulp fibers can be easily separated from the top-surface sheet, the back-surface sheet, and the pulp fibers. Therefore, the constituent members of sanitary product can be efficiently separated and collected.

In the present embodiment, the separation step S13 may further include a second separation step S33 of removing the adhesive of the bonding portion with a solvent that dissolves the adhesive of the bonding portion between the film and the other members. In the present embodiment, the adhesive of each bonding portion is removed with a solvent that dissolves the adhesive of each bonding portion between the film, the nonwoven fabric, and the absorbent body.

In the second separation step S33, the adhesive of the bonding portion between the film (back-surface sheet) and other members (nonwoven fabric of the top-surface sheet, top-surface sheet, absorbent body remaining on the surface of the back-surface sheet, and the like) is removed with the solvent. Due to that, the film and other members can be separated from each other while maintaining the same shape without breaking or the like. Therefore, the constituent members such as the film of sanitary product can be efficiently collected. In addition, since the film and other members can be separated without remaining the adhesive on the film, the film can be reused as a resin having high purity. Due to that, it is possible to suppress the adhesive from adversely affecting the film when it is reused. The same applies to the nonwoven fabric as well as the film.

The solvent used in the second separation step S33 is not particularly limited as long as it can dissolve the adhesive, and for example, a terpene containing at least one of terpene hydrocarbon, terpene aldehyde, and terpene ketone is used. In this step, an aqueous solution containing terpene is used, and a concentration of terpene in the aqueous solution is, for example, 0.05 mass % to 2 mass %. It is preferably 0.075 to 1 mass %. In a case where the concentration of terpene is extremely low, the adhesive of the bonding portion may not be able to be dissolved. In a case where the concentration of terpene is extremely high, the cost may increase. In addition, the terpene not only dissolves an adhesive such as a hot-melt adhesive, but also has an oil dirt cleaning effect. Therefore, for example, in a case where the constituent members of the sanitary product such as the back-surface sheet have printing, the terpene can also decompose and remove the printing ink.

Examples of terpene hydrocarbon include myrcene, limonene, pinene, camphor, sabinene, phellandrene, paracimene, ocimene, terpinene, kalen, zingiberene, caryophyllene, bisabolene, and cedrene. Among these, limonene, pinene, terpinene, and kalen are preferable. In addition, examples of the terpene aldehyde include citronellal, citral, cyclocitral, safranal, phellandral, perillaldehyde, geranial, and neral. Examples of the terpene ketone include camphor and thujone. Among the terpenes, terpene hydrocarbon is preferable and limonene is particularly preferable. There are three types of limonene which are d-limonene, 1-limonene, and dipentene (dl-limonene), and any of them can be preferably used. Terpene can be used alone or used in combination of two or more types thereof.

A treatment temperature of the second separation step S33, that is, a temperature of the aqueous solution containing the solvent is not particularly limited as long as the dissolution of the adhesive proceeds and the used sanitary product are decomposed into constituent members. The treatment temperature may be room temperature or higher than room temperature, and is, for example 15 to 30°C. In addition, a treatment time of the second separation step S33, that is, a time for immersing the top-surface sheet, the back-surface sheet, and the absorbent body in the aqueous solution containing the solvent is not particularly limited as long as the dissolution of the adhesive proceeds and the used sanitary product are decomposed into constituent members. The treatment time is, for example, 2 to 60 minutes and preferably 5 to 30 minutes. An amount of the aqueous solution of the second separation step S33, that is, an amount of the aqueous solution containing the solvent is not particularly limited as long as the dissolution of the adhesive proceeds and the used sanitary product are decomposed into constituent members. The amount of the aqueous solution is, for example, preferably 300 to 3,000 parts by mass and more preferably 500 to 2,500 parts by mass, with respect to 100 parts by mass of the used sanitary product. By the second separation step S33, the amount of the adhesive remaining on the film, the nonwoven fabric, the absorbent body, and the like can be set to 1 mass % or less with respect to the film, the nonwoven fabric, the absorbent body, and the like.

In the present embodiment, as another preferred embodiment, the second separation step S33 may be performed together with the inactivation step S31. That is, the adhesive attached to the top-surface sheet, the back-surface sheet, and the pulp fibers may be dissolved while inactivating the superabsorbent polymers attached to the top-surface sheet, the back-surface sheet, and the pulp fibers. In this case, as the aqueous solution for immersing the top-surface sheet, the back-surface sheet, the pulp fibers and the superabsorbent polymers, an aqueous solution containing both the inactivating agent and the solvent is used. Due to that, in the inactivation step S31, the back-surface sheet (film), the top-surface sheet (nonwoven fabric), and the absorbent body (pulp fibers and superabsorbent polymers) can be substantially separated in the aqueous solution. Then, in the subsequent first separation step, the back-surface sheet (film) and the top-surface sheet (nonwoven fabric) can be separated from the absorbent body (pulp fibers and superabsorbent polymers), and the second separation step S33 can be omitted. In this case, the back-surface sheet (film) and the top-surface sheet (nonwoven fabric) are substantially separated by removing the adhesive.

In the present embodiment, the separation step S13 may further include a first drying step S34 for drying the film with atmosphere or hot air at a temperature higher than room temperature to remove the solvent, after the step of removing the adhesive of the bonding portion. In the present embodiment, the nonwoven fabric is also dried in this step.

The sterilization is extremely important in the reuse of the used sanitary product. In the first drying step S34, a step of drying the separated film (back-surface sheet) and nonwoven fabric (top-surface sheet) in the atmosphere at high temperature or hot air is performed. A drying temperature is, for example, 105°C to 210°C and preferably 110°C to 190°C. A drying time depends on the drying temperature, and is, for example, 10 to 120 minutes and preferably 15 to 100 minutes. Due to that, not only the solvent remaining on the surface of the film and the nonwoven fabric is evaporated and removed, but also the film and the nonwoven fabric can be sterilized by the atmosphere or hot air at high temperature. Due to that, it is possible to exhibit a sterilizing (disinfecting) effect while removing the solvent.

On the other hand, in the present embodiment, the separation step S13 may include a third separation step S35 of separating the pulp fibers from the separated mixture. In the third separation step S35, the method of separating the pulp fibers from the separated mixture (including the pulp fibers, the superabsorbent polymers, and the wastewater) is not particularly limited, and for example, the separated mixture is discharged while passing through a screen having an opening of 0.1 to 4 mm, preferably an opening of 0.15 to 2 mm. Due to that, the pulp fibers can be separated from the mixture by remaining the superabsorbent polymers and the wastewater in the sewage and remaining the pulp fibers (superabsorbent polymers remains mainly on surface) on the screen. These pulp fibers contain a large amount of impurities, and they can be reused in this state depending on the purpose. The superabsorbent polymers are attached to the separated pulp fibers, and the separated pulp fibers and the superabsorbent polymers attached to the pulp fibers are mixed with water at a predetermined ratio, to obtain pulp fibers-containing material, and the process proceeds to an ozone treatment step S36.

In the present embodiment, the separation step S13 includes the ozone treatment step S36 of treating the pulp fibers-containing material containing the superabsorbent polymers, the pulp fibers, the coupling structure thereof, and water with an aqueous solution containing ozone, reducing a molecular weight of the superabsorbent polymers attached to the pulp fibers for solubilization and removal.

In the used sanitary product, in an absorbent body, etc., containing pulp fibers and superabsorbent polymers, (i) the superabsorbent polymers enlarge as they absorb a liquid such as a bodily fluid and entrains the pulp fibers, (ii) the enlarged superabsorbent polymers generate gel blocking while entraining the pulp fibers,etc., and accordingly, the plurality of superabsorbent polymers and the plurality of pulp fibers may form a coupling structure. The pulp fibers-containing material includes, in addition to the free pulp fibers and the free superabsorbent polymers, a coupling structure configured with a plurality of superabsorbent polymers and a plurality of pulp fibers.

In the ozone treatment step S36, the superabsorbent polymers contained in the pulp fibers-containing material (treatment liquid) are oxidatively decomposed by ozone in the aqueous solution and solubilized in the aqueous solution to be removed. The state in which the superabsorbent polymers are oxidatively decomposed and solubilized in the aqueous solution refers to a state in which the superabsorbent polymers and the coupling structure pass through a 2 mm screen. Due to that, impurities such as the superabsorbent polymers can be removed from the pulp fibers-containing material (treatment liquid) to produce a high-purity pulp fibers. In addition, secondary sterilization, bleaching, and deodorization of pulp fibers can be performed by the ozone treatment.

Fig. 2 is a schematic view showing a reference example of a configuration of an apparatus 2 which executes a reference example ozone treatment step S36. The apparatus 2 includes pulp fibers-containing material storage unit 3 which stores pulp fibers-containing material 51 containing water, the pulp fibers separated in the third separation step S35, and the superabsorbent polymers, and an ozone treatment unit 4 which removes the superabsorbent polymers contained in the pulp fibers-containing material 51 from the pulp fibers by oxidative decomposition.

The pulp fibers-containing material storage unit 3 includes pulp fibers-containing material tank 12 and a stirrer 13. The pulp fibers-containing material tank 12 stores the pulp fibers-containing material 51 supplied via a pipe 61. The stirrer 13 stirs the pulp fibers-containing material 51 in the pulp fibers-containing material tank 12 so that the pulp fibers and the superabsorbent polymers in the pulp fibers-containing material 51 are not separated from water and not precipitated to a lower portion of the pulp fibers-containing material 51.

Meanwhile, the ozone treatment unit 4 includes a supply pump 21, a treatment tank 31, an ozone supply device 41, a delivery pump 22, and an ozone decomposition device 34. The treatment tank 31 contains an acidic aqueous solution as a treatment liquid 52. The treatment tank 31 includes pulp fibers-containing material supply port 32, a treatment liquid discharge port 33, and an ozone-containing gas supply port 43. The pulp fibers-containing material supply port 32 is arranged on an upper part of the treatment tank 31 and supplies the pulp fibers-containing material 51 to the treatment tank 31. The treatment liquid discharge port 33 is arranged on a lower part of the treatment tank 31 and discharges the treatment liquid 52. The ozone-containing gas supply port 43 is arranged on a lower part of the treatment tank 31, specifically, on an upper part of the treatment liquid discharge port 33, and delivers the ozone-containing gas 53 into the treatment tank 31.

Specifically, the supply pump 21 continuously supplies the pulp fibers-containing material 51 of the pulp fibers-containing material tank 12 into the treatment tank 31 from the pulp fibers-containing material supply port 32 at a first flow rate via the pipe 62. The ozone supply device 41 supplies the ozone-containing gas 53 to the treatment tank 31. Examples of an ozone generation device 42 of the ozone supply device 41 include an ozone water exposure tester ED-OWX-2 manufactured by Ecodesign Co., Ltd., an ozone generation device OS-25V manufactured by Mitsubishi Electric Corporation, and the like. The ozone-containing gas 53 is another type of gas containing ozone, and examples thereof include an oxygen gas containing ozone. The ozone-containing gas supply port 43 delivers the ozone-containing gas 53 supplied to the treatment tank 31 via the pipe 65 into the treatment tank 31, and is arranged on a lower part (preferably the bottom portion) of the treatment tank 31. The ozone-containing gas supply port 43 continuously supplies the ozone-containing gas 53 as a plurality of fine bubbles into the treatment liquid 52 from a lower part to an upper part of the treatment liquid 52 (treatment tank 31). The delivery pump 22 continuously discharges the treatment liquid 52 in the treatment tank 31 from the treatment liquid discharge port 33 to the outside of the treatment tank 31 at a second flow rate via the pipe 63. The ozone decomposition device 34 receives the ozone-containing gas 53 accumulated in the upper part of the treatment tank 31 via the pipe 64, and detoxifies and release the ozone to the outside. In addition, the treatment liquid 52 in the treatment tank 31 is only the treatment liquid 52 before the start of the ozone treatment step S36, and is a liquid obtained by mixing the treatment liquid 52 and the pulp fibers-containing material 51 after the start thereof. The liquid in the treatment tank 31 including the liquid obtained by mixing the treatment liquid 52 and the pulp fibers-containing material 51 is defined as the treatment liquid 52.

Next, a specific method of the ozone treatment step S36 will be described. The pulp fibers and the superabsorbent polymers separated in the third separation step S35 are mixed with water so as to have a preset concentration to obtain the pulp fibers-containing material 51. The concentration of the pulp fibers in the pulp fibers-containing material 51 is set to be the preset concentration in a state in which it is put to the treatment tank 31 and mixed with the treatment liquid 52. The pulp fibers-containing material 51 is supplied to and stored in the pulp fibers-containing material tank 12 via a pipe 61. Since the specific gravities of the pulp fibers and the superabsorbent polymers are greater than 1, the pulp fibers-containing material 51 is stirred with the stirrer 13 in the pulp fibers-containing material tank 12 so that the pulp fibers and the superabsorbent polymers do not separate from water.

The flow rate of the pulp fibers-containing material 51 in the pulp fibers-containing material tank 12 is controlled by the supply pump 21, and the pulp fibers-containing material 51 is continuously supplied from the pulp fibers-containing material supply port 32 to the treatment tank 31 via the pipe 62 at the first flow rate. The treatment liquid 52 is an acidic aqueous solution and has a specific gravity of approximately 1. Therefore, the pulp fibers and the superabsorbent polymers are precipitated from the upper part to the lower part of the treatment liquid 52.

Meanwhile, the ozone-containing gas 53 generated by the ozone generation device 42 is supplied to the treatment tank 31 via the pipe 65 and released in a state of fine bubbles (for example, microbubbles or nanobubbles) into the treatment liquid 52 from the ozone-containing gas supply port 43 of the treatment tank 31. That is, the ozone-containing gas 53 rises from the lower part to the upper part of the treatment liquid 52.

Then, in the treatment liquid 52, the pulp fibers and the superabsorbent polymers which move downward, that is, fall, and the ozone-containing gas 53 which moves upward, that is, rises, proceed to face each other and collide with each other. The ozone-containing gas 53 is attached to the surfaces of the pulp fibers, the superabsorbent polymers, and the coupling structure. The ozone in the ozone-containing gas 53 oxidatively decomposes the free superabsorbent polymers and dissolves it in the treatment liquid 52. Due to that, the superabsorbent polymers on the pulp fibers are removed from the pulp fibers. The pulp fibers fall to the bottom portion of the treatment tank 31, and the ozone-containing gas 53 is released to a space on the upper part of the treatment tank 31.

In the free superabsorbent polymers, the free pulp fibers, and the coupling structure, the free superabsorbent polymers and coupling structure containing the superabsorbent polymers having a relatively low buoyancy tends to have higher sedimentation properties than the free pulp fibers having a relatively high buoyancy. Meanwhile, the ozone-containing gas rises while consuming ozone and treating the superabsorbent polymers and the pulp fibers, and accordingly, the ozone-containing gas existing at the lower position has a higher ozone content ratio than the ozone-containing gas existing at an upper position (that is, it tends to be fresh).

Accordingly, the free superabsorbent polymers and the coupling structure which move downward relatively quickly, can be accurately oxidatively decomposed with the fresher ozone-containing gas to form free pulp fibers. Meanwhile, since the free pulp fibers moves downward relatively slowly, the ozone-containing gas can treat the free pulp fibers and the pulp fibers for cellulose nanofiberization to be formed by taking time. Specifically, the ozone in the ozone-containing gas faces and collides with the pulp fibers, and accordingly, lignin of the pulp fibers (and pulp fibers for cellulose nanofiberization) can be decomposed.

After that, the treatment liquid 52 (including pulp fibers for cellulose nanofiberization) on the bottom portion of the treatment tank 31 is continuously discharged to outside of the treatment tank 31 from the treatment liquid discharge port 33 of the treatment tank 31 via the pipe 63 at the second flow rate, by controlling the flow rate of the delivery pump 22. The ozone of the ozone-containing gas 53 accumulated on the upper part of the treatment tank 31 is detoxified by the ozone decomposition device 34 and released to the outside.

As described above, the pulp fibers-containing material 51 is continuously supplied into the treatment tank 31 from the upper part of the treatment tank 31 at the first flow rate, and the treatment liquid 52 is continuously discharged from the lower part (bottom portion) of the treatment tank 31 to the outside of the treatment tank 31 at the second flow rate. Due to that, a continuous and stable flow of fluid (including pulp fibers) from the upper part to the lower part can be forcibly generated in the treatment tank 31.

The treatment liquid 52 discharged from the treatment tank 31 contains pulp fibers for cellulose nanofiberization, from which the superabsorbent polymers have been removed, and contains a low molecular weight organic material generated by oxidative decomposition of the superabsorbent polymers. The pulp fibers for cellulose nanofiberization are collected in a step at the downstream side of the delivery pump 22, for example, in a fourth separation step S37 which will be described later.

In this method, at least the pulp fibers-containing material 51 containing the pulp fibers and the superabsorbent polymers is continuously supplied into the treatment tank 31 containing the treatment liquid 52 capable of dissolving the superabsorbent polymers at the first flow rate, and the treatment liquid 52 containing the pulp fibers for cellulose nanofiberization, from which the superabsorbent polymers have been removed, and containing a low molecular weight organic material generated by the oxidative decomposition of the superabsorbent polymers is continuously discharged to outside of the treatment tank 31 at the second flow rate. With the configuration described above, a continuous and stable flow of fluid (including pulp fibers) can be forcibly generated from the pulp fibers-containing material supply port 32 for supplying the pulp fibers-containing material 51 in the treatment tank 31 toward the treatment liquid discharge port 33 for discharging the treatment liquid 52. With the flow of the fluid, that is, a water flow, the superabsorbent polymers can be treated (solubilized) and the pulp fibers can be treated, even in a case where an amount of treatment of the pulp fibers and the superabsorbent polymers is increased.

Here, it is preferable that the first flow rate and the second flow rate are the same. By setting the first flow rate and the second flow rate to be the same, the amount of the treatment liquid 52 in the treatment tank 31 can be maintained constant, and stable and continuous treatment can be realized. However, in a case where the amount of the treatment liquid 52 in the treatment tank 31 can be maintained substantially constant, that is, unless the amount of the treatment liquid 52 in the treatment tank 31 is significantly increased or decreased, the first flow rate and the second flow rate may fluctuate over time. That is, the first flow rate and the second flow rate do not have to be completely the same at all times, and may be substantially the same on average over time. Here, the expression, substantially the same means that a difference between the first flow rate and the second flow rate is within 5 mass %. In this case as well, stable and continuous treatment can be realized.

In a case where the ozone-containing gas 53 is supplied to the treatment liquid 52, an ozone concentration in the treatment liquid 52 is not particularly limited as long as it can oxidatively decompose the superabsorbent polymers, and is, for example, 1 to 50 mass ppm, preferably 2 to 40 mass ppm, and more preferably 3 to 30 mass ppm. In a case where the ozone concentration in the treatment liquid 52 is extremely low, there is a risk that the superabsorbent polymers cannot be completely solubilized, and the superabsorbent polymers may remain in the pulp fibers. On the other hand, in a case where the ozone concentration in the treatment liquid 52 is extremely high, an oxidizing power is also increased, which may damage the pulp fibers and may cause a problem in safety. An ozone treatment temperature is not particularly limited as long as it can oxidatively decompose the superabsorbent polymers, and may be kept at room temperature or may be higher than room temperature, for example.

The concentration of ozone in the treatment liquid 52 (aqueous solution) is measured by the following method.
(1) In a 100 mL graduated cylinder containing approximately 0.15 g of potassium iodide and 5 mL of a 10% citric acid solution, 85 mL of the treatment liquid 52 in which ozone is dissolved is put and reacted.
(2) The treatment liquid 52 after the reaction is moved to a 200 mL Erlenmeyer flask, a starch solution is added into the Erlenmeyer flask, the mixture is colored in purple, and then titrated while stirring until it turns to be colorless with 0.01 mol/L sodium thiosulfate, and the added amount a (mL) is recorded.
(3) The concentration of ozone in the aqueous solution is calculated using the following equation. The concentration of ozone in the aqueous solution (mass ppm) is calculated by the following equation: concentration of ozone in the aqueous solution (mass ppm) = a (mL) × 0.24 × 0.85 (mL).

The ozone concentration in the ozone-containing gas 53 is preferably 40 to 200 g/m³, more preferably 40 to 150 g/m³, and even more preferably 40 to 100 g/m³. In a case where the ozone concentration in the ozone-containing gas 53 is extremely low, there is a risk that the superabsorbent polymers cannot be completely solubilized, and the superabsorbent polymers may remain. In a case where the concentration in the ozone-containing gas 53 is extremely high, it may cause damage to pulp fibers, a deterioration in safety, and an increase in production cost. The ozone concentration in the ozone-containing gas 53 can be measured by, for example, an ultraviolet absorption type ozone concentration meter (for example, manufactured by Ecodesign Co., Ltd.: ozone monitor OZM-5000G).

The concentration of the pulp fibers and superabsorbent polymers in the treatment liquid 52 is not particularly limited as long as it is a concentration at which the superabsorbent polymers can be oxidatively decomposed by ozone in the treatment liquid 52 and is, for example, 0.1 to 20 mass %, preferably 0.2 to 10 mass %, and more preferably 0.3 to 5 mass %. In a case where the concentration of the pulp fibers is extremely high, there is a risk that the superabsorbent polymers cannot be completely solubilized, and the superabsorbent polymers may remain in the pulp fibers. On the other hand, in a case where the concentration of the pulp fibers is extremely low, an oxidizing power is also increased, which may damage the pulp fibers and may cause a problem in safety. The concentrations of the pulp fibers and the superabsorbent polymers in the pulp fibers-containing material 51 are appropriately set based on the concentrations of the pulp fibers and the superabsorbent polymers in the treatment liquid 52 and the amount of the treatment liquid 52.

In a case where the ozone is supplied to the treatment liquid 52 containing the pulp fibers and the superabsorbent polymers, the treatment liquid 52 is preferably acidic (specifically, pH of more than 0.0 and pH of less than 3.0, preferably pH of 2.5 or more and pH of less than 3.0), weakly acidic (specifically, pH of 3.0 or more and pH of less than 6.0), or neutral (specifically, pH of 6.0 or more and pH of 8.0 or less, preferably pH of 6.0 or more and pH of 7.0 or less). The pH of the treatment liquid 52 is more preferably more than 0.0 and 7.0 or less, and even more preferably 2.5 to 6.0. By treating in an acidic state, the deactivation of ozone is suppressed, the oxidative decomposition effect of the superabsorbent polymers by ozone is enhanced, and the superabsorbent polymers can be oxidatively decomposed in a short time. In order to maintain the pH of the treatment liquid, the pH of the pulp fibers-containing material 51 may be set to be the same as the pH of the treatment liquid 52, and the pulp fibers-containing material 51 may be supplied to the treatment tank 31. Alternatively, the pH of the treatment liquid 52 may be monitored with a pH sensor, and in a case where the pH fluctuates to the neutral side, a predetermined acidic solution may be added to the treatment liquid 52 by an amount corresponding to a fluctuation range.

The amount of the treatment liquid 52 (including the pulp fibers-containing material 51) in the treatment tank 31 is not particularly limited as long as the superabsorbent polymers can be oxidatively decomposed, and it is preferable that a volume V (unit: L) of the treatment liquid 52 in the treatment tank 31 and a mass W (unit: kg) of the pulp fibers satisfy 30 ≦ V/W ≦ 1000. These more preferably satisfy 50 ≦ V/W ≦ 400, and even more preferably satisfy 100 ≦ V/W ≦ 200. In a case where the V/W is extremely small, there is a risk that the superabsorbent polymers cannot be completely solubilized, and the superabsorbent polymers remain. In a case where the V/W is extremely large, there is a risk that the production cost increases. The volume V of the treatment tank 31 is not particularly limited, and is, for example, 50 to 80 L.

A flow rate R₀ (Unit: L/min) of the ozone-containing gas and the volume V (unit: L) of the treatment liquid 52 in treatment tank 31 preferably satisfy 0.01 ≦ R₀ /V ≦ 1.25. These more preferably satisfy 0.03 ≦ R₀/V ≦ 1.0 and even more preferably satisfy 0.06 ≦ R₀/V ≦ 0.75. In a case where R₀/V is extremely small, there is a risk that the superabsorbent polymers cannot be completely solubilized, and the superabsorbent polymers may remain in the pulp fibers. In a case where R₀/V is extremely large, it may cause damage to pulp fibers, a deterioration in safety, and an increase in production cost. The flow rate R₀ of the ozone-containing gas is not particularly limited, and is, for example, 3 to 6L/min.

The time during which the pulp fibers are present in the treatment tank 31, that is, the time during which the pulp fibers are treated in the treatment liquid 52 (hereinafter, also referred to as "in-tank treatment time") is not particularly limited, as long as it is time during which the superabsorbent polymers can be oxidatively decomposed. The in-tank treatment time may be short, in a case where the ozone concentration of the treatment liquid 52 is high, and may be long, in a case where the ozone concentration of the treatment liquid 52 is low. The in-tank treatment time is, for example, 2 minutes to 60 minutes and preferably 5 minutes to 30 minutes. The product of the ozone concentration (mass ppm) in the treatment liquid 52 and the in-tank treatment time (minutes) (hereinafter, also referred to as a "CT value") is preferably 100 to 6,000 ppm min, more preferably 200 to 4,000 ppm.min, and even more preferably 300 to 2,000 ppm.min. In a case where the CT value is extremely small, there is a risk that the superabsorbent polymers cannot be completely solubilized, and the superabsorbent polymers may remain in the collected pulp fibers. In a case where the CT value is extremely large, it may cause damage to pulp fibers, a deterioration in safety, and an increase in production cost.

While the pulp fibers are present in the treatment tank 31, the superabsorbent polymers are oxidatively decomposed into low molecular weight components by ozone and dissolved in the treatment liquid 52. The low molecular weight component dissolved in the treatment liquid 52 is discharged together with the treatment liquid 52. In addition, in this step, the used sanitary product is primarily disinfected by the sterilization action of the ozone.

In the present reference example, the ozone treatment step S36 (continuous treatment step) includes a step of continuously discharging the treatment liquid 52 from the lower part of the treatment tank 31 while continuously supplying the pulp fibers-containing material 51 from the upper part of the treatment tank 31. The specific gravity of the pulp fibers and the superabsorbent polymers in the pulp fibers-containing material 51 is greater than the specific gravity of water of the treatment liquid 52, and accordingly, the pulp fibers, the superabsorbent polymers, and the coupling structure are naturally precipitated.

In the present reference example, the treatment liquid 52 capable of dissolving the superabsorbent polymers is an aqueous solution containing an ozone-containing gas that oxidatively decomposes the superabsorbent polymers so as to be soluble. The ozone treatment step S36 (continuous treatment step) further includes a delivery step of continuously delivering a plurality of bubbles of the ozone-containing gas from the lower part to the upper part of the treatment liquid 52. In one reference example of the method, in the treatment liquid 52, the ozone-containing gas rises, and the pulp fibers and the superabsorbent polymers fall, that is, have countercurrent. Due to that, a contact probability between the pulp fibers and the superabsorbent polymers, and the ozone-containing gas can be increased. In addition, the deeper the pulp fibers and superabsorbent polymers are precipitated, the higher the concentration of ozone-containing gas can be contacted. Therefore, the superabsorbent polymers, which could not be completely dissolved in the treatment liquid 52 only by the ozone-containing gas coming into contact with a shallow part of the treatment liquid 52, can come into contact with the high-concentration ozone-containing gas in the treatment liquid 52 in the deep part. Due to that, the superabsorbent polymers can be reliably dissolved in the treatment liquid 52. Therefore, the superabsorbent polymers can be reliably dissolved in the treatment liquid and removed from the fiber.

In a preferable embodiment, the delivery step described above includes a step of delivering the ozone-containing gas in a state of microbubbles or nanobubbles. However, the microbubbles are bubbles having a diameter of approximately 1 to 1,000 µm and preferably approximately 10 to 500 µm, and the nanobubbles are bubbles having a diameter of approximately 100 to 1000 nm, preferably approximately 100 to 500 nm. The microbubbles or nanobubbles are fine bubbles as described above, have properties of a large surface area per unit volume, and a slow speed of rise in the liquid. Therefore, in this method, as a preferable embodiment, the ozone-containing gas containing the fine bubbles described above is delivered from the lower part to the upper part of the treatment liquid 52 in the treatment tank 31.

Meanwhile, in the present reference example of Figure 2, the pulp fibers and the superabsorbent polymers move from the upper part to the lower part. At this time, since the raising speed of the fine bubbles is slow, the probability that the bubbles come into contact with the pulp fibers can be increased. In addition, since the fine bubbles occupy a small area on the surface of the pulp fibers, more bubbles can come into contact with the surface of the pulp fibers. Due to that, the pulp fibers, the superabsorbent polymers, and the coupling structure can be evenly wrapped with fine bubbles, and a contact area between them and the ozone-containing gas can be further increased. In addition, more bubbles come into contact with the surface of the pulp fibers, and accordingly, the sedimentation properties of the pulp fibers, the superabsorbent polymers, and the coupling structure can be decreased by the buoyancy of the bubbles, and the contact time between them and the ozone-containing gas can be increased. Therefore, the superabsorbent polymers can be more reliably dissolved in the treatment liquid 52 and removed from the pulp fibers.

In a preferable embodiment, the treatment liquid 52 is an acidic aqueous solution and is, for example, an acidic aqueous solution having a pH of 2.5 or less. In that case, even in a case where the superabsorbent polymers in the pulp fibers-containing material 51 partially have a remaining water absorption capacity, the water absorption expansion of the superabsorbent polymers can be suppressed. Due to that, the superabsorbent polymers can be dissolved in the treatment liquid 52 in a short time, and the superabsorbent polymers can be removed more reliably. In particular, in a case where the treatment liquid 52 is an ozone-containing aqueous solution, the ozone in the ozone-containing aqueous solution is less likely to be deactivated, and accordingly, the superabsorbent polymers can be oxidatively decomposed and dissolved in a shorter time, and the superabsorbent polymers can be more reliably removed from the fiber.

In addition, as another reference example, the configuration of the treatment tank 31 may have a configuration other than that shown in Fig. 2. Fig. 3 is a schematic view showing another reference example of the apparatus 2 of an ozone treatment step. The apparatus 2 of Fig. 3 is different from the apparatus 2 of Fig. 2, in that the pipe 63 of the ozone treatment unit 4 has a continuous U-shaped tube structure in which two U-shaped tubes are continuously connected upside down and the delivery pump 22 is omitted. In that case, in a case where the pipe 63 is filled with the treatment liquid 52 and a height of a liquid level of the treatment liquid 52 in the treatment tank 31 is higher than a height of a liquid level of the liquid in the tank of the next step connected by the pipe 63, the treatment liquid 52 is discharged to the tank in the next step via the pipe 63 according to the principle of siphon. Accordingly, in a case where the height of the liquid level of the treatment liquid 52 in the treatment tank 31 is set to be the same as the height of the liquid level of the liquid in the tank of the next step initially before the start of the treatment, when the pulp fibers-containing material 51 is continuously supplied into the treatment tank 31 at the first flow rate by the start of the treatment, the treatment liquid 52 is discharged to the tank in the next step via the pipe 63 at the second flow rate = the first flow rate according to the principle of siphon. However, regarding the height of the liquid level of the liquid in the tank in the next step, the height before the start of the treatment is maintained even during the treatment. In this case, the delivery pump 22 is not necessary, and the control of the second flow rate of the delivery pump 22 is not necessary.

In one embodiment, the separation step S13 further includes a fourth separation step S37 of separating the pulp fibers from the treatment liquid 52 discharged from the treatment tank 31, and a second drying step S38 of drying the separated pulp fibers.

In the fourth separation step S37, the method of separating the pulp fibers from the treatment liquid 52 discharged from the treatment tank 31 is not particularly limited, and for example, a method of causing the treatment liquid 52 containing the pulp fibers for cellulose nanofiberization to pass through a screen mesh having an opening of 0.15 to 2 mm is used. In a case where the treatment liquid 52 containing the pulp fibers for cellulose nanofiberization is passed through a screen mesh having an opening of 0.15 to 2 mm, wastewater containing a product produced by oxidative decomposition of the superabsorbent polymers passes through the screen. Meanwhile, the pulp fibers for cellulose nanofiberization remains on the screen.

In the subsequent second drying step S38, the separated pulp fibers are dried with an atmosphere at a high temperature or hot air. A drying temperature is, for example, 105°C to 210°C and preferably 110°C to 190°C. A drying time depends on the drying temperature, and is, for example, 10 to 120 minutes and preferably 15 to 100 minutes. Due to that, the solvent remaining on the surface of the pulp fibers is evaporated and removed, and pulp fibers having extremely low superabsorbent polymers content and high purity can be collected. Therefore, the constituent members for sanitary product can be efficiently collected. In addition, the pulp fibers can be sterilized (disinfected) in the atmosphere at a high temperature or hot air.

In the present disclosure, the pulp fibers for cellulose nanofiberization have a lignin content ratio of 0.1 mass % or less, preferably 0.08 mass % or less, and more preferably 0.06 mass % or less. By doing so, the pulp fibers for cellulose nanofiberization are excellent in cellulose nanofiberization property.

The lignin content ratio of pulp fibers for cellulose nanofiberization can be measured based on a method disclosed in pages 85 to 87 of "Soil Diagnosis Guide Description Method" published on March 1988 by the Agricultural Guidance Division of the Agriculture, Forestry and Fisheries Department of Ehime Prefecture. The outline is as below.

### <Preparation of Reagent>

(1) Pulp fibers for cellulose nanofiberization dried at 120°C for 60 minutes are prepared and left for 24 hours in a constant temperature and constant humidity chamber with a temperature of 20 ± 5°C and a humidity of 65 ± 5% RH.
(2) Commercially available lignin (95%) is prepared as a standard reagent.
(3) 22.3 g of sodium pyrophosphate (Na₄P₂O₅ · 10H₂O) is weighed in a 500 mL beaker, approximately 400 mL of deionized water is added to dissolve sodium pyrophosphate, and a sodium pyrophosphate aqueous solution is prepared.
(4) 10 g of sodium hydroxide is weighed in another 500 mL beaker, approximately 200 mL of deionized water is added to dissolve sodium hydroxide, and a sodium hydroxide aqueous solution is prepared. The sodium pyrophosphate aqueous solution and the sodium hydroxide aqueous solution are allowed to cool and added to a 1 L volumetric flask, and the volume is adjusted with deionized water to prepare a pyrophosphate extract.

### < Pre-Operation >

(5) 2 g of lignin is weighed in a 100 mL Erlenmeyer flask A.

(6) 2 g of the pulp fibers for cellulose nanofiberization is weighed in another 100 mL Erlenmeyer flask B.

(7) 20 mL of pyrophosphate extract is added to each of the Erlenmeyer flask A and the Erlenmeyer flask B and shaken for 3 minutes.

(8) After allowing the Erlenmeyer flask A and the Erlenmeyer flask B to stand for 15 minutes, the filtering is performed with No. 6 filter paper to obtain a lignin filtrate and pulp fibers filtrate for cellulose nanofiberization.

### <Analysis>

(9) Using a whole pipette, 5 mL of the lignin filtrate is added to a 50 mL volumetric flask and a volume thereof is adjusted with deionized water (lignin 10,000 ppm).

(10) Using a measuring pipette, 1 mL, 2 mL, 5 mL, and 10 mL of the lignin filtrate with adjusted volume are added to four 100 mL volumetric flasks, respectively, and the deionized water is added to adjust the volume, and a calibration liquid (100 ppm, 200 ppm, 500 ppm, and 1,000 ppm) is formed.

(11) Deionized water was used as a blank, a transmittance of the calibration liquid is measured at a wavelength of 530 nm and converted to an absorbance with reference to the absorbance conversion table on page 235.

(12) A calibration curve of the calibration liquid concentration and the absorbance is created.

(13) The transmittance of the pulp fibers filtrate for cellulose nanofiberization is measured using deionized water as a blank, converted to absorbance with reference to the absorbance conversion table on page 235, and the lignin concentration is calculated from the calibration curve.

(14) The lignin content ratio (mass %) is calculated from the lignin concentration. The commercially available lignin (95%) described above may be changed to commercially available lignin such as lignin concentration (for example, lignin manufactured by Nacalai Tesque Inc.)

In the production method of the present disclosure, the pulp fibers for cellulose nanofiberization have a beating degree reduction rate of preferably 300 mL or more, more preferably 320 mL or more, even more preferably 340 mL or more, and still more preferably 360 mL or more. By doing so, the pulp fibers for cellulose nanofiberization are easily fluffed and easily converted into cellulose nanofibers in the subsequent cellulose nanofiber forming step.

In the production method of the present disclosure, the pulp fibers for cellulose nanofiberization have a beating degree reduction rate of preferably 990 mL or less, more preferably 800 mL or less, even more preferably 700 mL or less, and still more preferably 600 mL or less. By doing so, it is possible to suppress damage on the pulp fibers for cellulose nanofiberization and the cellulose nanofiber to be produced. The beating degree reduction rate is obtained by achieving a low lignin content ratio of the pulp fibers for cellulose nanofiberization, a narrow distribution of lignin content ratio, and the like by performing the production method of the present disclosure.

The beating degree reduction rate is measured according to the following beating degree reduction test.

### <beating degree reduction test>

(1) The pulp fibers for cellulose nanofiberization are beaten for 1 hour or longer, preferably 2 hours according to the pulp-beating method-Part 1: beater method of JIS P8221-1: 1998.
(2) Samples are collected every 20 minutes after the start of beating, and the beating degree of each sample (Canadian standard freeness) is measured according to the pulp-freeness test method-Part 2: Canadian standard freeness method of JIS P8121-2: 2012. The test may be stopped in a case where the beating degree of the sample is less than 100 mL.
(3) The time (h) is plotted on a horizontal axis and the beating degree (mL) is plotted on a vertical axis, and approximated to a linear function by the least squares method, and an absolute value of a slope is used as the beating degree reduction rate (mL/m).

A large value of the beating degree reduction rate means that the reduction in beating degree per unit time is fast, that is, the pulp fibers for cellulose nanofiberization are easily beaten (easily fluffed).

In the present disclosure, the pulp fibers for cellulose nanofiberization have a water contact angle of preferably 20° or less, more preferably 15° or less, and even more preferably 10° or less. By doing so, the pulp fibers for cellulose nanofiberization produced by the production method described above can be easily dispersed in an aqueous solution, for the conversion to the cellulose nanofiber after being dried and stored. From the above viewpoint, the water contact angle of the pulp fibers for cellulose nanofiberization may be 0°.

The water contact angle of the pulp fibers for cellulose nanofiberization can be measured as follows.
(1) An aluminum ring (outer diameter: 43 mm, inner diameter: 40 mm, height: 5 mm) and the pulp fibers for saccharification dried at 120°C for 60 minutes are prepared and left for 24 hours in a constant temperature and constant humidity chamber with a temperature of 20 ± 5°C and a humidity of 65 ± 5% RH.
(2) 1.5 g of the pulp fibers for cellulose nanofiberization is evenly filled in the aluminum ring, and the pulp fibers for cellulose nanofiberization are compressed together with the aluminum ring at a pressure of 3 Mpa for 1 minute using a press machine with a smooth bottom face and the surface of the pulp fibers for cellulose nanofiberization is smoothed.
(3) The water contact angle of the compressed pulp fibers for cellulose nanofiberization is measured based on 6. sessile drop method of "Method of testing the wettability of the substrate glass surface" of JIS R3257: 1999. As a contact angle measuring device, an automatic contact angle meter CA-V type manufactured by Kyowa Interface Science Co., Ltd. is used. The water contact angle means a value after 200 ms after dropping deionized water.
(4) The water contact angle is measured in 20 different samples, and an average value thereof is used.

In the present disclosure, the pulp fibers for cellulose nanofiberization have an ash content of preferably 0.65 mass % or less, more preferably 0.50 mass % or less, even more preferably 0.30 mass %, and still preferably 0.20 mass % or less. Therefore, in a case of producing cellulose nanofibers from the pulp fibers for cellulose nanofiberization, metal ions and their precipitates are less likely to damage equipment used in cellulose nanofibers forming step, and it is difficult to inhibit miniaturization of the pulp fibers for cellulose nanofiberization. The ash content can be lowered by selecting an acid capable of forming a complex with a metal ion contained in excrement as an inactivating agent, particularly a citric acid, in the inactivation step S31 of inactivating the superabsorbent polymers.

In the present specification, the ash content means the amount of an inorganic or nonflammable residue left after the organic matter is ashed, and the ash content means the ratio (mass ratio) of the ash content contained in the material to be promoted. The ash content is measured according to "5. Ash content test method" of "2. General test method" of the material standard for menses treatment products. Specifically, the ash content is measured as follows.
(1) A platinum, quartz, or porcelain crucible is preheated at 500°C to 550°C for 1 hour, allowed to cool, and then the mass thereof is precisely weighed.
(2) 2 to 4 g of the pulp fibers for cellulose nanofiberization dried at 120°C for 60 minutes are collected and put in a crucible, and the mass thereof is weighed precisely, it is weakly heated at first by removing or shifting a lid of the crucible, if necessary, and the temperature is gradually increased to heat at 500°C to 550°C for 4 hours or longer to ash until no carbide remains.
(3) After allowing to cool, the mass is weighed precisely. The residue is ashed again to a constant weight, allowed to cool, and then the mass thereof is precisely weighed to obtain the ash content (mass %).

In a preferred embodiment, the method further includes the inactivation step S31 of inactivating absorption performance of the superabsorbent polymers in the mixture by treating a mixture using the aqueous solution capable of inactivating the absorption performance of the superabsorbent polymers before the ozone treatment step S36 (continuous treatment step), and the first separation step S32 of separating the inactivated superabsorbent polymers and the pulp fibers from the aqueous solution before the ozone treatment step S36 (continuous treatment step). As described above, in the present method, as a preferred embodiment, in the inactivation step S31, the water absorption performance of the superabsorbent polymers is suppressed by the aqueous solution capable of inactivating the water absorption performance of the superabsorbent polymers, and accordingly, at the stage of ozone treatment step S36 (continuous treatment step) which is the subsequent step, the superabsorbent polymers can be more easily dissolved by the treatment liquid 52 in a short time.

In the present embodiment, as a preferred embodiment, in the inactivation step S31, the aqueous solution capable of inactivating the water absorption performance of the superabsorbent polymers is an acidic aqueous solution, for example, an acidic aqueous solution having a pH of 2.5 or less. As described above, in the present method, as a preferred embodiment, since the aqueous solution capable of inactivating the absorption performance of the superabsorbent polymers is an acidic aqueous solution, the superabsorbent polymers are more easily inactivated, and accordingly, at the stage of the inactivation step S31, the absorption performance of the superabsorbent polymers can be suppressed more reliably. Due to that, the superabsorbent polymers can be more easily dissolved by the treatment liquid for a short time at the stage of the ozone treatment step S36 (continuous treatment step) which is the subsequent step.

In addition, as another preferred embodiment, the treatment tank 31 may include at least a first treatment tank 31-1 and a second treatment tank 31-2 coupled in series with each other. Fig. 4 is a schematic view showing another reference example of an apparatus 2 of a reference example ozone treatment step. The apparatus 2 of Fig. 4 is different from the apparatus 2 of Fig. 2 in that two ozone treatment units 4 are bonded in series, in other words, the first treatment tank 31-1 and the second treatment tank 31-2 are bonded in series. In that case, for example, the first treatment tank 31-1 supplies the pulp fibers-containing material 51 and discharges a first treated liquid (treatment liquid 52-1 of the first treatment tank 31-1), and the second treatment tank 31-2 supplies the first treated liquid and discharges a second treated liquid (treatment liquid 52-2 of the second treatment tank 31-2), thereby treating the pulp fibers-containing material 51 at multiple stages. In that case, compared to the case where one treatment tank 31 having a large capacity is provided, the treatment is performed with new treatment liquids 52-1 and 52-2 for each of the first and second treatment tanks 31-1 and 31-2, and accordingly, for example, the superabsorbent polymers that could not be completely dissolved in the first treatment tank (first stage treatment tank) 31-1 can be easily dissolved in the second treatment tank (next stage treatment tank) 31-2, and accordingly, the superabsorbent polymers can be dissolved more reliably and can be removed from the fiber.

In a preferred embodiment, in the material separation step S1, in the pretreatment step S11, the used sanitary product is kept in the same shape without breaking and the superabsorbent polymers are not inactivated and can be expanded extremely with water. Due to that, an extremely high internal pressure can be generated in the used sanitary product, and any portion of the surface thereof can be in a state where it is likely to be burst. Then, in the decomposition step S12, by applying a physical impact to the used sanitary product in such a state, any portion of the surface thereof is torn and the internal absorbent core can be ejected to the outside. Due to that, the used sanitary product can be decomposed into at least a film (back-surface sheet) and an absorbent core. At this time, since the film generally maintains its original shape, it can be easily separated from the absorbent core in the subsequent separation step S13. Due to that, the constituent member such as the film can be separated from other constituent members while maintaining the same shape without breaking or the like. Therefore, the constituent members such as the film for sanitary product can be efficiently collected.

In the present embodiment, as a preferred embodiment, by using terpene for removing the adhesive, the hot-melt adhesive for adhering the constituent members of the sanitary product can be dissolved at room temperature. Due to that, the sanitary product can be easily and neatly separated, the pulp fibers and the superabsorbent polymers can be separated from the sanitary product, and the nonwoven fabric and the film can be separated separately while leaving the member form. That is, the pulp fibers, the film, and the nonwoven fabric can be easily collected separately without crushing the sanitary product or going through a complicated separation step. In a case where limonene is used as a terpene, as a side effect of limonene, there is a refreshing citrus odor, and accordingly, the odor derived from excrement can be covered to some extent, and the burden of odor on the operator and the influence of odor on the neighborhood can be reduced. Limonene is a monoterpene and is similar in structure to styrene, and accordingly, a styrene-based hot-melt adhesive commonly used in sanitary product can be dissolved. Since the sanitary product can be cleaned at room temperature, energy costs can be reduced and the generation and diffusion of odors can be suppressed. Terpene has a high oil dirt cleaning effect, and in addition to the effect of dissolving hot-melt adhesive, in a case where there is printing on the film, the printing ink can also be decomposed and removed, and the printed film can also be collected as a high-purity plastic material.

In addition, in a case where an organic acid aqueous solution having a pH of 2.5 or less is used for inactivating the superabsorbent polymers, the pulp fibers are difficult to be deteriorated. In a case where a citric acid is used as the organic acid, the effect of removing dirt components derived from excrement can be expected due to the chelating effect and detergency of citric acid. In addition, a sterilizing effect and a deodorizing effect on alkaline odors can be expected.

In addition, by oxidatively decomposing the superabsorbent polymers with ozone, it is possible to prevent contamination of pulp fibers and a rapid increase in wastewater due to water absorption of the superabsorbent polymers. By adjusting the ozone concentration, it is possible to simultaneously perform oxidative decomposition and sterilization of superabsorbent polymers. In addition, in a case where the ozone is used, no chlorine-based chemicals are used, and accordingly it is possible to produce high-quality RPF that does not easily damage a combustion furnace from the collected plastic members. Since no salts are used during the treatment step, there is no residue on the pulp fibers, and high-quality pulp fibers for cellulose nanofiberization with low ash content can be collected.

The production method of the present disclosure can further include cellulose nanofibers forming step of forming cellulose nanofibers from the pulp fibers for cellulose nanofiberization, after the treatment liquid discharge step. The cellulose nanofiber forming step is not particularly limited, and includes a well-known cellulose nanofiber forming method in the technical field. Examples of the cellulose nanofiber forming method include methods disclosed in JP-A-2010-235681 and JP-A-2010-254726.

In the cellulose nanofiber forming step, as shown in Fig. 1, the dried pulp fibers for cellulose nanofiberization obtained in the second drying step S38 can be subjected to the cellulose nanofiber formation step, the undried pulp fibers for cellulose nanofiberization obtained in the fourth separation step S37 may be subjected to the cellulose nanofiber formation step, or the treatment liquid 52 including the pulp fibers for cellulose nanofiberization obtained in the ozone treatment step S36 can be subjected to the cellulose nanofiber formation step.

### EXAMPLES

Pulp fibers for cellulose nanofiberization was produced from a plurality of types of used disposable diapers collected from nursing facility according to the methods shown in Figs. 1 and 2. The conditions related to the ozone treatment step S36 were as follows.
(i) Pulp fibers-containing material 51
   - Concentration: 1 mass % (concentration of pulp fibers and superabsorbent polymers)
   - pH: 2.4
(ii) Treatment tank 31
   - Capacity: 60L
   - Height: 2.6m
   - First flow rate: 2 L/min
   - Second flow rate: 2 L/min
   - In-tank treatment time: 30 minutes
   - V/W: 100
   - R₀/V: 0.033
(iii) Ozone-containing gas
   - Ozone concentration: 100 g/m³
   - Shape: Nanobubbles

### [Manufacturing Example 1]

The inactivation step S31 was carried out with a citric acid having a pH of 2.0, the ozone treatment step S36 was carried out under the conditions described above, and the obtained pulp fibers for cellulose nanofiberization was dried at 120°C for 60 minutes to obtain pulp fibers No. 1 for cellulose nanofiberization.

### [Manufacturing example 2]

Pulp fibers No. 2 for cellulose nanofiberization was carried out in the same manner as in Manufacturing Example 1, except that the inactivation step S31 was carried out with slaked lime.

### [Comparative Manufacturing Example 1]

Pulp fibers No. 3 for cellulose nanofiberization was carried out in the same manner as in Manufacturing Example 1, except that the ozone-containing gas was not delivered in the ozone treatment step S36.

### [Comparative Manufacturing Example 2]

Pulp fibers No. 4 for cellulose nanofiberization was carried out in the same manner as in Manufacturing Example 2, except that the ozone-containing gas was not delivered in the ozone treatment step S36.

### [Comparative Manufacturing Example 3]

NBKP virgin pulp fibers was used as pulp fibers No. 5 for cellulose nanofiberization.

### [Examples 1 and 2 and Comparative Examples 1 to 3]

A lignin content ratio (mass %), a water contact angle (°), and an ash content (mass %) of each of the pulp fibers No. 1 to No. 5 for cellulose nanofiberization were measured according to the methods described in the present specification. The results are indicated in Table 1. A beating degree reduction rate (mL/h) and a beating degree (mL) after beating for a predetermined period of time in a beating degree reduction test of each of the pulp fibers No. 1, No. 2, and No. 5 for cellulose nanofiberization were measured according to the beating degree reduction test disclosed in the present specification. The results are also shown in Table 1.

### [Table 1]

**Table1**

| Example No. | Example 1 | Example 2 | Comparative Example 1 | Comparative Example 2 | Comparative Example 3 |
|---|---|---|---|---|---|
| Pulp fiber No. for cellulose nanofiberization | No.1 | No.2 | No.3 | No.4 | No.5 |
| Inactivation | Acid | Slaked lime | Acid | Slaked lime | - |
| Ozone treatment | Performed | Performed | Not performed | Not performed | - |
| Lignin content (mass %) | <0.10 | <0.10 | 0.10 | 0.15 | 0.16 |
| Water contact angle | 0 | 0 | 83.1 | -^{a)} | 13.9 |
| Ash content (mass %) | 0.09 | 0.15 | 0.16 | 16 | 0.12 |
| Beating degree reduction rate (mL/h) | 485 | 360 | - | - | 275 |
| Beating degree (mL)/time (min) | 88/80 | 138/120 | - | - | 270/120 |

| | | | | | |
|---|---|---|---|---|---|
| a) Not measurable due to a large amount of foreign materials | | | | | |

### [REFERENCE SIGNS LIST]

31 treatment tank
32 pulp fibers-containing material supply port
33 treatment liquid discharge port
43 ozone-containing gas supply port
51 pulp fibers-containing material
52 treatment liquid
53 ozone-containing gas
S36 ozone treatment step

## Claims

1. A method of producing pulp fibers for cellulose nanofiberization from pulp fibers contained in a used sanitary product, the method comprising:
a preparation step of preparing a treatment tank (31) including a pulp fibers-containing material supply port (32), a treatment liquid discharge port (33), and an ozone-containing gas supply port (43) arranged at a lower position of the treatment tank (31);
a pulp fibers-containing material supply step of supplying pulp fibers-containing material (51) to the treatment tank (31) from the pulp fibers-containing material supply port (32);
an ozone-containing gas supply step of supplying ozone-containing gas (53) to a treatment liquid (52) in the treatment tank (31) from the ozone-containing gas supply port (43);
a pulp fibers for cellulose nanofiberization forming step of forming the pulp fibers for cellulose nanofiberization having a lignin content ratio of 0.1 mass % or less, measured according to the method described in the description, from the pulp fibers, by supplying the ozone-containing gas (53) to the treatment tank (31) containing the treatment liquid (52) containing the pulp fibers-containing material (51) containing superabsorbent polymers and the pulp fibers which derive from the used sanitary product, as well as bringing the pulp fibers-containing material (51) into contact with the ozone-containing gas (53) while raising the ozone-containing gas (53) in the treatment tank (31), to dissolve at least a part of the superabsorbent polymers in the treatment liquid (52); and
a treatment liquid discharge step of discharging the treatment liquid (52) containing the pulp fibers for cellulose nanofiberization from the treatment liquid discharge port (33),
**characterized in that** the treatment liquid discharge port (33) is arranged on an upper side of the pulp fibers-containing material supply port (32), and in the pulp fibers for cellulose nanofiberization forming step, the pulp fibers-containing material (51) is brought into contact with the ozone-containing gas (53) while raising the pulp fibers-containing material (51).

2. The method according to claim 1, further comprising an inactivation step of inactivating the superabsorbent polymers with an acid before the pulp fibers-containing material supply step.

3. The method according to claim 2, wherein the acid is an acid capable of forming a complex with a metal ion contained in excrement.

4. The method according to any one of claims 1 to 3,
wherein, in the pulp fibers-containing material supply step, the pulp fibers-containing material (51) is continuously supplied from the pulp fibers-containing material supply port (32) to the treatment tank (31) at a first flow rate, and in the treatment liquid discharge step, the treatment liquid (52) is continuously discharged from the treatment liquid discharge port (33) at a second flow rate.

5. The method according to any one of claims 1 to 4, wherein the treatment liquid (52) is acidic, weakly acidic, or neutral.

6. The method according to any one of claims 1 to 5, wherein, in the pulp fibers for cellulose nanofiberization forming step, the ozone-containing gas (53) is supplied as microbubbles or nanobubbles.

7. The method according to any one of claims 1 to 6, further comprising cellulose nanofibers forming step of forming cellulose nanofibers from the pulp fibers for cellulose nanofiberization, after the pulp fibers for cellulose nanofiberization forming step.

8. The method according to any one of claims 1 to 7, wherein the pulp fibers for cellulose nanofiberization have a beating degree reduction rate of 300 mL/h or more, measured according to the method described in the description.

9. The method according to any one of claims 1 to 8, wherein the pulp fibers for cellulose nanofiberization have an ash content of 0.65 mass % or less.

## Patentansprüche

1. Verfahren zum Produzieren von Zellstofffasern für Zellulosenanofaserisierung aus Zellstofffasern, die in einem gebrauchten Hygieneartikel enthalten sind, wobei das Verfahren umfasst:
einen Vorbereitungsschritt des Vorbereitens eines Behandlungsbehälters (31), der eine zellstofffaserhaltige Materialzufuhröffnung (32), eine Ausstoßöffnung (33) für eine Behandlungsflüssigkeit und eine ozonhaltige Gaszufuhröffnung (43), die in einer niedrigeren Position des Behandlungsbehälters (31) angeordnet ist;
einen Zuführschritt von zellstofffaserhaltigem Material des Zuführens von zellstofffaserhaltigem Material (51) aus der zellstofffaserhaltigen Materialzufuhröffnung (32) an den Behandlungsbehälter (31);
einen Zuführschritt von ozonhaltigem Gas des Zuführens von ozonhaltigem Gas (53) an eine Behandlungsflüssigkeit (52) in dem Behandlungsbehälter (31) von der ozonhaltigen Gaszufuhröffnung (43);
einen Ausbildungsschritt für Zellstofffasern für Zellulosenanofaserisierung des Ausbildens der Zellstofffasern für Zellulosenanofaserisierung mit einem Ligningehaltsverhältnis von 0,1 Masse-% oder weniger, gemessen gemäß dem in der Beschreibung beschriebenen Verfahren, aus den Zellstofffasern, indem das ozonhaltige Gas (53) an den Behandlungsbehälter (31), der die Behandlungsflüssigkeit (52) enthält, die das zellstofffaserhaltige Material (51) enthält, das superabsorbierende Polymer und Zellstofffasern enthält, die aus dem gebrauchten Hygieneartikel gewonnen werden, zugeführt wird sowie das das zellstofffaserhaltige Material (51) mit dem ozonhaltigen Gas (53) in Berührung gebracht wird, während das ozonhaltige Gas (53) in dem Behandlungsbehälter (31) angehoben wird, um wenigstens einen Teil der superabsorbierenden Polymer in der Behandlungsflüssigkeit (52) aufzulösen; und
einen Ausstoßschritt der Behandlungsflüssigkeit des Ausstoßens der Behandlungsflüssigkeit (52), die die Zellstofffasern für die Zellulosenanofaserisierung enthält, aus der Ausstoßöffnung (33) für die Behandlungsflüssigkeit,
**dadurch gekennzeichnet, dass** die Ausstoßöffnung (33) für die Behandlungsflüssigkeit auf einer oberen Seite der zellstofffaserhaltigen Materialzufuhröffnung (32) angeordnet ist und das zellstofffaserhaltige Material (51) in dem Ausbildungsschritt für Zellstofffasern für Zellulosenanofaserisierung mit dem ozonhaltigen Gas (53) in Berührung gebracht wird, während das zellstofffaserhaltige Material (51) angehoben wird.

2. Verfahren nach Anspruch 1, ferner umfassend einen Inaktivierungsschritt des Inaktivierens der superabsorbierenden Polymer mit einer Säure vor dem zellstofffaserhaltigen Materialzufuhrschritt.

3. Verfahren nach Anspruch 2, wobei die Säure eine Säure ist, die dazu in der Lage ist, einen Komplex mit einem Metallion, das in den Fäkalien enthalten ist, auszubilden.

4. Verfahren nach einem der Ansprüche 1 bis 3,
wobei in dem zellstofffaserhaltigen Materialzufuhrschritt das zellstofffaserhaltige Material (51) kontinuierlich von der zellstofffaserhaltigen Materialzufuhröffnung (32) an den Behandlungsbehälter (31) mit einer ersten Durchflussrate zugeführt wird und in dem Ausstoßschritt für die flüssige Behandlung die Behandlungsflüssigkeit (52) kontinuierlich aus der Ausstoßöffnung (33) für die Behandlungsflüssigkeit mit einer zweiten Durchflussrate ausgestoßen wird.

5. Verfahren nach einem der Ansprüche 1 bis 4, wobei die Behandlungsflüssigkeit (52) sauer, schwach sauer oder neutral ist.

6. Verfahren nach einem der Ansprüche 1 bis 5, wobei in dem Ausbildungsschritt der Zellstofffasern für die Zellulosenanofaserisierung das ozonhaltige Gas (53) als Mikrobläschen oder Nanobläschen zugeführt wird.

7. Verfahren nach einem der Ansprüche 1 bis 6, ferner umfassend den Schritt des Ausbildens von Zellulosenanofasern aus den Zellstofffasern für die Zellulosenanofaserisierung nach dem Ausbildungsschritt von Zellstofffasern für die Zellulosenanofaserisierung.

8. Verfahren nach einem der Ansprüche 1 bis 7, wobei die Zellstofffasern für die Zellulosenanofaserisierung eine Schlaggradreduktionsrate von 300 ml/h oder mehr aufweisen, gemessen gemäß dem in der Beschreibung beschriebenen Verfahren.

9. Verfahren nach einem der Ansprüche 1 bis 8, wobei die Zellstofffasern für die Zellulosenanofaserisierung einen Aschegehalt von 0,65 Massen-% oder weniger aufweisen.

## Revendications

1. Procédé permettant de produire des fibres de pâte à papier à des fins de nanodéfibrage de cellulose en provenance de fibres de pâte à papier contenues dans un produit sanitaire usagé, le procédé comportant :
une étape de préparation consistant à préparer un réservoir de traitement (31) comprenant un orifice d'alimentation en matière contenant des fibres de pâte à papier (32), un orifice de décharge de liquide de traitement (33), et un orifice d'alimentation en gaz contenant de l'ozone (43) agencé au niveau d'une position inférieure du réservoir de traitement (31) ;
une étape d'alimentation en matière contenant des fibres de pâte à papier consistant à alimenter de la matière contenant des fibres de pâte à papier (51) jusque dans le réservoir de traitement (31) en provenance de l'orifice d'alimentation en matière contenant des fibres de pâte à papier (32) ;
une étape d'alimentation en gaz contenant de l'ozone consistant à alimenter du gaz contenant de l'ozone (53) jusque dans un liquide de traitement (52) dans le réservoir de traitement (31) en provenance de l'orifice d'alimentation en gaz contenant de l'ozone (43) ;
une étape de formation de fibres de pâte à papier à des fins de nanodéfibrage de cellulose consistant à former les fibres de pâte à papier à des fins de nanodéfibrage de cellulose ayant un rapport de teneur en lignine de 0,1 % en masse ou moins, que l'on mesure en fonction du procédé décrit dans la description, en provenance des fibres de pâte à papier, en fournissant le gaz contenant de l'ozone (53) jusque dans le réservoir de traitement (31) contenant le liquide de traitement (52) contenant la matière contenant des fibres de pâte à papier (51) contenant des polymères superabsorbants et les fibres de pâte à papier que l'on dérive du produit sanitaire usagé, ainsi qu'en mettant la matière contenant des fibres de pâte à papier (51) en contact avec le gaz contenant de l'ozone (53) tout en faisant monter le gaz contenant de l'ozone (53) dans le réservoir de traitement (31), pour dissoudre au moins une partie des polymères superabsorbants dans le liquide de traitement (52) ; et
une étape de décharge de liquide de traitement consistant à décharger le liquide de traitement (52) contenant les fibres de pâte à papier à des fins de nanodéfibrage de cellulose en provenance de l'orifice de décharge de liquide de traitement (33),
**caractérisé en ce que** l'orifice de décharge de liquide de traitement (33) est agencé sur un côté supérieur de l'orifice d'alimentation en matière contenant des fibres de pâte à papier (32), et au cours de l'étape de formation de fibres de pâte à papier à des fins de nanodéfibrage de cellulose, la matière contenant des fibres de pâte à papier (51) est mise en contact avec le gaz contenant de l'ozone (53) tout en faisant monter la matière contenant des fibres de pâte à papier (51).

2. Procédé selon la revendication 1, comportant par ailleurs une étape d'inactivation consistant à inactiver les polymères superabsorbants avec un acide avant l'étape d'alimentation en matière contenant des fibres de pâte à papier.

3. Procédé selon la revendication 2, dans lequel l'acide est un acide en mesure de former un complexe avec un ion métallique contenu dans les excréments.

4. Procédé selon l'une quelconque des revendications 1 à 3, dans lequel, au cours de l'étape d'alimentation en matière contenant des fibres de pâte à papier, la matière contenant des fibres de pâte à papier (51) est alimentée en continu en provenance de l'orifice d'alimentation en matière contenant des fibres de pâte à papier (32) jusque dans le réservoir de traitement (31) à un premier débit d'écoulement, et au cours de l'étape de décharge de liquide de traitement, le liquide de traitement (52) est déchargé en continu en provenance de l'orifice de décharge de liquide de traitement (33) à un deuxième débit d'écoulement.

5. Procédé selon l'une quelconque des revendications 1 à 4, dans lequel le liquide de traitement (52) est acide, faiblement acide ou neutre.

6. Procédé selon l'une quelconque des revendications 1 à 5, dans lequel, au cours de l'étape de formation de fibres de pâte à papier à des fins de nanodéfibrage de cellulose, le gaz contenant de l'ozone (53) est fourni sous la forme de microbulles ou de nanobulles.

7. Procédé selon l'une quelconque des revendications 1 à 6, comportant par ailleurs une étape de formation de nanofibres de cellulose consistant à former des nanofibres de cellulose à partir des fibres de pâte à papier à des fins de nanodéfibrage de cellulose, après l'étape de formation de fibres de pâte à papier à des fins de nanodéfibrage de cellulose.

8. Procédé selon l'une quelconque des revendications 1 à 7, dans lequel les fibres de pâte à papier à des fins de nanodéfibrage de cellulose ont un taux de réduction de degré de raffinage de 300 mL/h ou plus, que l'on mesure en fonction du procédé décrit dans la description.

9. Procédé selon l'une quelconque des revendications 1 à 8, dans lequel les fibres de pâte à papier à des fins de nanodéfibrage de cellulose ont une teneur en cendres de 0,65 % en masse ou moins.
